# EUROPEAN PATENT APPLICATION

(11) **EP 4 026 897 A1**
(43) Date of publication of application: **13.07.2022**
(21) Application number: 20860174.0
(22) Date of filing: 02.09.2020
(51) Int. Cl.: C12N 5/071, C12N 11/02

(54) **METHOD FOR PRODUCING FROZEN BODY OF THREE-DIMENSIONAL TISSUE AGGREGATE OF RETINAL PIGMENT EPITHELIAL CELLS**

(30) Priority: 02.09.2019 JP 2019159895
(71) Applicant: Nichirei Biosciences Inc., Chuo-ku Tokyo 104-8402 (JP); RIKEN, Wako-shi Saitama 351-0198 (JP)
(72) Inventor: INADA Ayaka, Sayama-shi, Saitama 350-1331 (JP); HOSOI Junko, Sayama-shi, Saitama 350-1331 (JP); TAKAHASHI Masayo, Wako-shi, Saitama 351-0198 (JP); KOIDE Naoshi, Wako-shi, Saitama 351-0198 (JP)
(74) Representative: TBK
(86) International application number: PCT/JP2020/033295
(87) International publication number: WO 2021/045122

(57) **Abstract**

The present disclosure provides a method for producing a frozen body of a three-dimensional tissue aggregate of retinal pigment epithelial cells, the method including a step of preparing the three-dimensional tissue aggregate of the retinal pigment epithelial cells, and a step of subjecting the three-dimensional tissue aggregate of the retinal pigment epithelial cells to a freezing treatment at a predetermined freezing temperature set in advance using, as an index, the occurrence rate of a linear aggregate of dead cells in the three-dimensional tissue aggregate of the retinal pigment epithelial cells after thawing, thus obtaining the frozen body.

## Description

### CROSS-REFERENCE TO RELATED APPLICATIONS

This application is based upon and claims the benefit of priority from Japanese Patent Application No. 2019-159895, filed on September 2, 2019; the entire contents of which are incorporated herein by reference.

### BACKGROUND OF THE INVENTION

### Field of the Invention

The present disclosure relates to a method for producing a frozen body of a three-dimensional tissue aggregate of retinal pigment epithelial cells.

### Background Art

A retinal pigment epithelium (RPE) cell sheet is a 3D tissue fabricated by culturing retinal pigment epithelial cells into a sheet-like form. Generally, the RPE cell sheet is fabricated by using a Transwell (trademark) insert. More specifically, it is possible to obtain the RPE cell sheet by culturing RPE cells in a maintenance medium and fabricating a collagen gel, which is a scaffold, in the insert, and then seeding the RPE cells on the collagen gel to culture them for usually about 2 months.

The RPE cell sheet is composed of monolayer cells, like the living body, and it has been confirmed that the sheet has an apico-basal polarity, which is characteristic of an epithelial tissue, and that a basement membrane is formed. A tight junction is formed between cells of the RPE cell sheet, and the RPE cell sheet can exhibit a barrier function such as transepithelial electric resistance (TER).

The RPE cell sheet is under development for the purpose of being used for autologous transplantation treatment of exudative age-related macular degeneration, which is one of eye diseases. In the case of autologous transplantation, it takes about 10 months to collect skin tissues from a patient and establish iPS cells therefrom, and induce differentiation into RPE cells and purify them to fabricate a sheet, and therefore preparation for transplantation requires vast amounts of time and costs.

Meanwhile, in the case of allogeneic transplantation in which an RPE cell sheet is fabricated from a specimen collected from a donor and then the sheet is transplanted, it is expected that the culture period until transplantation is shortened and that costs required for preparation are reduced. This merit can be obtained by stocking and preparing the RPE cell sheet fabricated in advance in the case of allogeneic transplantation. In order to stock the RPE cell sheet, it is necessary to establish a method in which the RPE cell sheet is stably cryopreserved for a long period of time.

In the method for cryopreserving cells, usually, the maintenance medium of cultured cells is removed and replaced with a preservation solution, followed by a freezing treatment. The preservation solution and the freezing method used are different for each subject, and particularly the freezing method is broadly divided into rapid freezing (vitrification freezing) and slow freezing.

The slow freezing method is a method in which cells are slowly frozen at a relatively high temperature (1°C/minute at -80°C). In slow freezing, since extracellular water is frozen first, a difference in osmotic pressure occurs between the intracellular and extracellular regions, and dehydration occurs due to the difference in osmotic pressure. Because of passing through the temperature at which ice is formed (maximum ice crystal formation zone) for a long period of time, extracellular ice crystals become larger during that period, possibly resulting in damage to cells. Regarding slow freezing, cytotoxicity due to a cryopreservation solution is low, while cells and tissues of a larger size cannot be completely dehydrated only in a cooling process, and thus slow freezing is generally considered as unsuitable for these cells and tissues.

Meanwhile, the rapid freezing method (vitrification freezing method) is a method in which cells are rapidly cooled using a freezing mixture such as liquid nitrogen and frozen (for example, 3600°C/minute for liquid nitrogen immersion). In vitrification freezing, cells are immersed in a cryopreservation solution at a high concentration, and intracellular water is instantaneously dehydrated to make the cells vitreous during freezing, and in addition, intracellular and extracellular regions are simultaneously frozen in a state in which no ice crystals are formed, and thus the cells are less likely to be damaged due to ice crystals. Therefore, the rapid freezing method is frequently used for freezing of tissues. Meanwhile, since rapid freezing uses a cryopreservation solution at a high concentration, cytotoxicity may be generally high. In order to prevent recrystallization, rapid thawing is also required, and skilled techniques are sometimes required.

In order to rapidly freeze cells, it is necessary to immerse the cells in a cryopreservation solution, dehydrate intracellular water, and concentrate the intracellular fluid. However, if dehydration of intracellular water or concentration of the intracellular fluid is insufficient, the intracellular water cannot be vitrified during freezing and is crystallized. If ice crystals are intracellularly formed, cell membranes and intracellular organs are damaged, and it becomes difficult to maintain the original cell state during thawing. Since intracellularly and extracellularly formed crystals grow during cryopreservation, the cell structure is also destroyed during the preservation period. Therefore, in order that the RPE cell sheet can maintain the structure equivalent to that before freezing even after thawing, it is necessary to establish a method for freezing by complete vitrification while avoiding recrystallization of water.

In recent years, in the field of regenerative medicine, various methods for cryopreserving cells have been reported. For example, Non-Patent Document 1 discloses cryopreservation of a two-dimensional monolayer tissue in which cells are treated with a preservation solution containing carboxylated polylysine, followed by vitrification using liquid nitrogen.

Patent Document 1 discloses a method for slowly vitrifying cultured cells in the form of a cell sheet or a three-dimensional cell culture, the method including a step of replacing a medium of cultured cells in the form of a cell sheet or a three-dimensional cell culture with a vitrification solution containing carboxylated ε -poly-L-lysine, and a step of cooling the cultured cells whose medium is replaced with the vitrification solution under a gaseous atmosphere at a temperature fall rate in the range of 0.08 to 1.00°C/second.

### Prior Art Document

### [Patent Document]

Patent Document 1: JP 6482052 B1

### [Non-Patent Document]

Non-Patent Document 1: Cryopreservation of a two-dimensional monolayer using a slow vitrification method with polyampholyte to inhibit ice crystal formation. K. Matsumura et.al., ACS Biomater. Sci. Eng. 2016, 2, 1023-1029

### SUMMARY OF THE INVENTION

However, the study by the present disclosers has revealed that, even when the methods mentioned in Patent Document 1 and Non-Patent Document 1 are applied to the RPE cell sheet, cell contraction and cell detachment are observed on the entire surface of the RPE cell sheet, and the sheet structure cannot be maintained.

Then, the present disclosers have further intensively studied, and this time found that, when a three-dimensional tissue aggregate of retinal pigment epithelial cells is subjected to a particular treatment, it is possible to obtain a frozen body that can be stably preserved while maintaining a high quality status.

Therefore, the present disclosure provides a method for producing a frozen body of a three-dimensional tissue aggregate of retinal pigment epithelial cells that can be stably preserved.

According to one embodiment of the present disclosure, provided is a method for producing a frozen body of a three-dimensional tissue aggregate of retinal pigment epithelial cells, the method including:
a step of preparing the three-dimensional tissue aggregate of the retinal pigment epithelial cells, and
a step of subjecting the three-dimensional tissue aggregate of the retinal pigment epithelial cells to a freezing treatment at a predetermined freezing temperature set in advance using, as an index, the occurrence rate of a linear aggregate of dead cells in the three-dimensional tissue aggregate of the retinal pigment epithelial cells after thawing, thus obtaining the above-mentioned frozen body.

According to the present disclosure, it is possible to stably produce a frozen body of a three-dimensional tissue aggregate of retinal pigment epithelial cells while maintaining high quality.

### BRIEF DESCRIPTION OF THE DRAWINGS

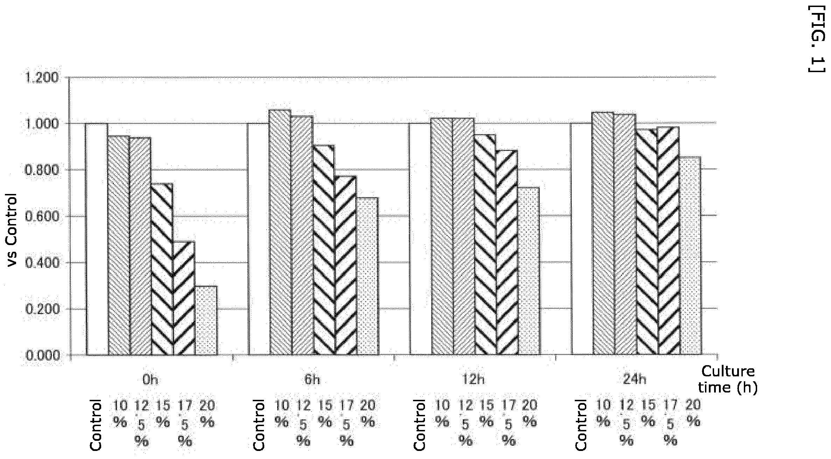

FIG. 1 is a graph showing the metabolic activity of cells when solutions having ethylene glycol concentrations of 10(v/v)%, 12.5(v/v)%, 15(v/v)%, 17.5(v/v)% and 20(v/v)% as pretreatment solutions were added to retinal pigment epithelial cells and the cells were cultured for 0 to 24 hours in Example 1.

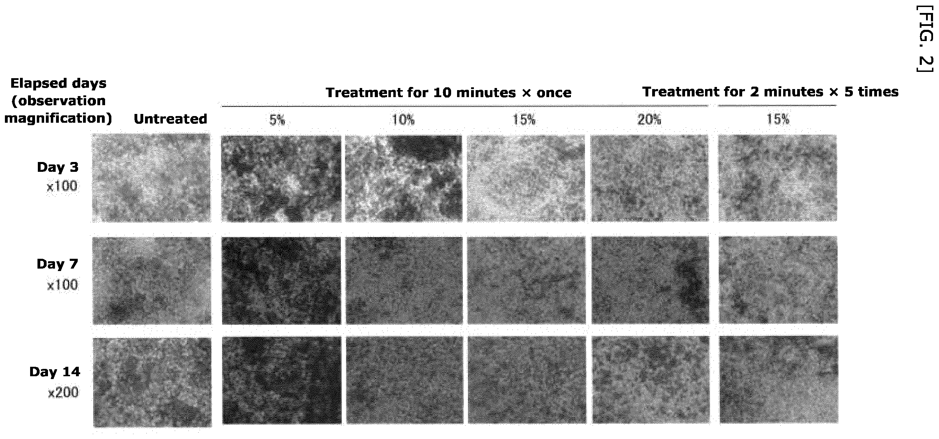

FIG. 2 shows photographs of untreated (pretreatment, freezing and thawing are not performed) RPE cell sheets, photographs of RPE cell sheets obtained by the single treatment method (pretreatment for 10 minutes × once; ethylene glycol concentration of 5, 10, 15 or 20(v/v)%), and photographs of RPE cell sheets obtained by the stepwise treatment method (pretreatment for 2 minutes × 5 times; ethylene glycol concentrations of 3, 6, 9, 12 and 15(v/v)%) in Example 2. Respective photographs were taken at the time points of days 3, 7 and 14 of culture after each treatment (observation magnification of ×100 for days 3 and 7 of culture, and observation magnification of x200 for day 14 of culture).

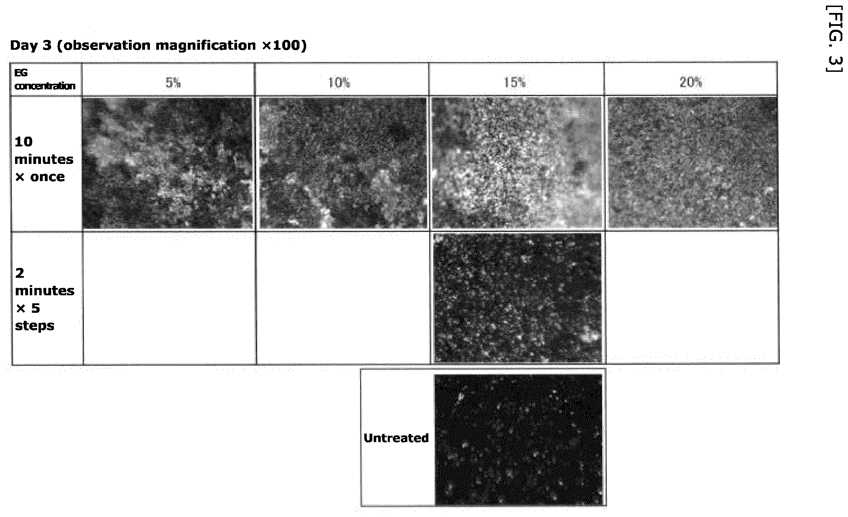

FIG. 3 shows photographs of SYTOX (trademark) Green staining by microscopy (observation magnification of ×100) in Example 2 (day 3 of culture). The upper photographs are micrographs of RPE cell sheets obtained by the single treatment method (pretreatment for 10 minutes × once; EG concentration of 5, 10, 15 or 20(v/v)%). The middle photograph is a micrograph of an RPE cell sheet obtained by the stepwise treatment method (pretreatment for 2 minutes × 5 times; EG concentration; 3, 6, 9, 12 and 15(v/v)%). The lower photograph is a micrograph of an untreated RPE cell sheet.

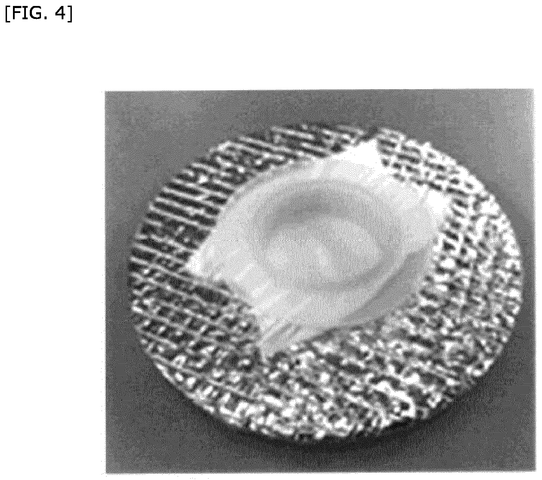

FIG. 4 shows a photograph of an apparatus (float for freezing) used when a laminate of a collagen gel and an RPE cell sheet was frozen using liquid nitrogen or thawed.

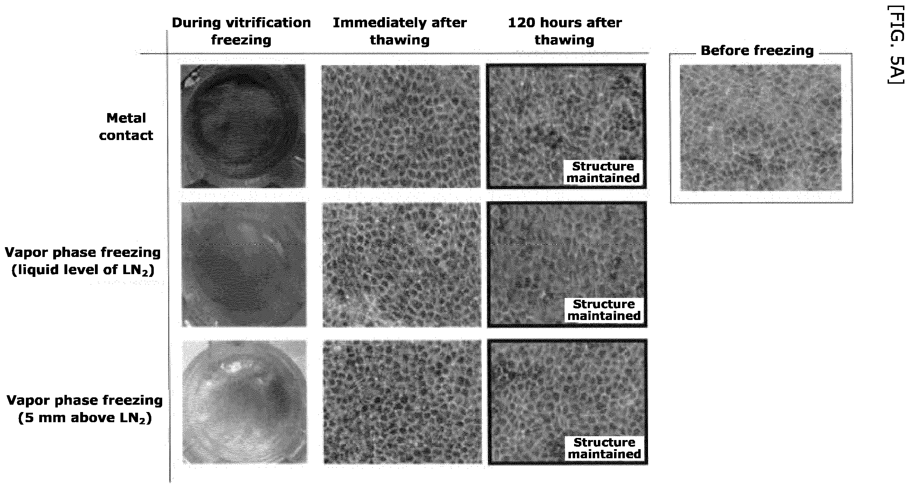

FIG. 5A shows photographs of RPE cell sheets subjected to a freezing treatment by metal contact (upper), vapor phase freezing (liquid level of liquid nitrogen) (middle) or vapor phase freezing (5 mm above the liquid level of liquid nitrogen) (lower) in Example 6. For respective samples, photographs during vitrification freezing, immediately after thawing, and 120 hours after thawing are shown. As a reference, a photograph of an RPE cell sheet before freezing is also shown.

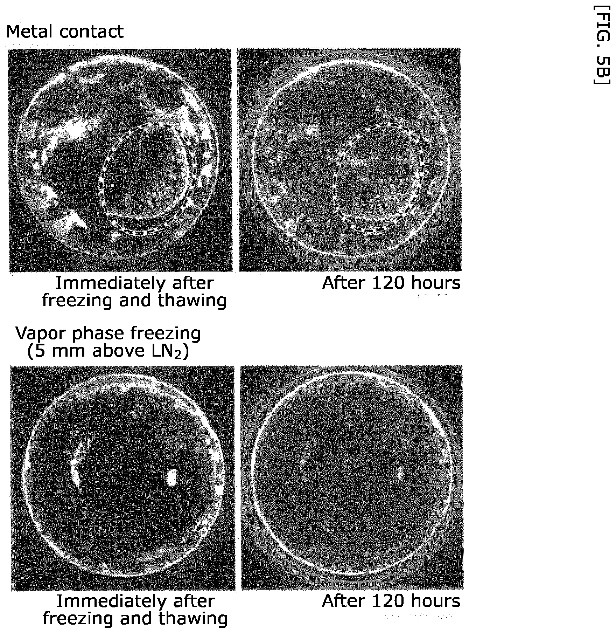

FIG. 5B shows photographs of RPE cell sheets subjected to a freezing treatment by metal contact (upper) or vapor phase freezing (5 mm above the liquid level of liquid nitrogen) (lower) and stained using SYTOX (trademark) Green in Example 6.

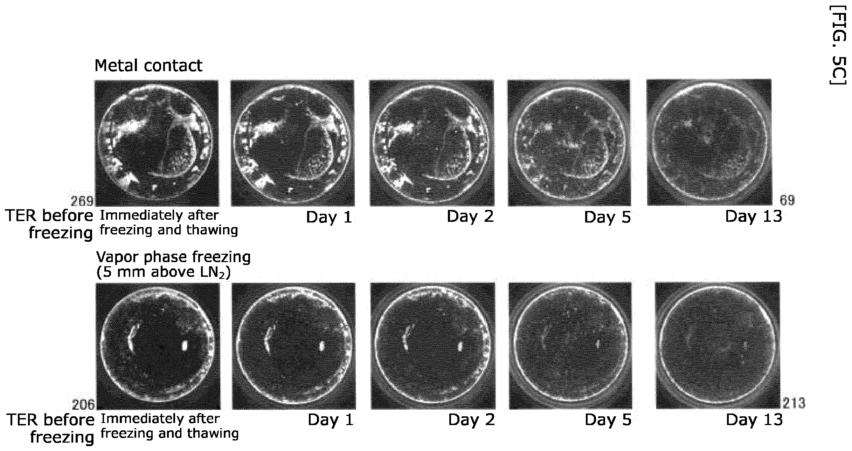

FIG. 5C shows photographs of RPE cell sheets subjected to a freezing treatment by metal contact (upper) or vapor phase freezing (5 mm above the liquid level of liquid nitrogen) (lower) and stained using SYTOX (trademark) Green and results of TER values in Example 6.

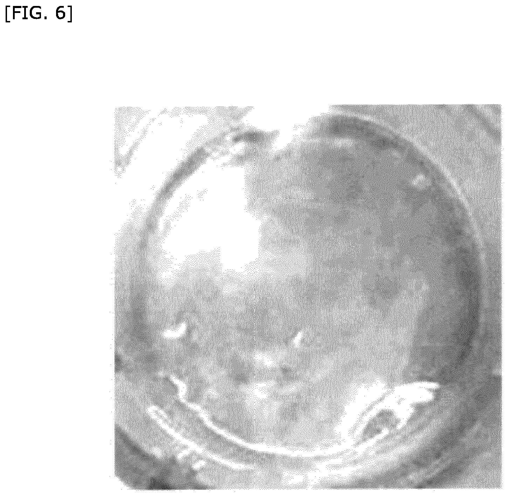

FIG. 6 shows a photograph of a laminate of a collagen gel and an RPE cell sheet that was completely vitrified obtained in Example 7 (7-3).

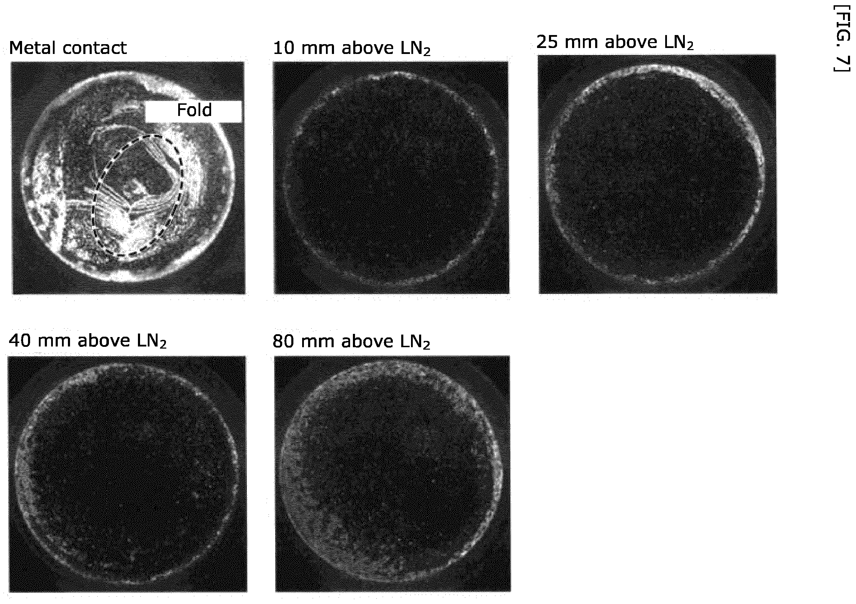

FIG. 7 shows photographs of SYTOX (trademark) Green staining after RPE cell sheets obtained by the metal contact method, vapor phase freezing (10 mm above the liquid level of liquid nitrogen), vapor phase freezing (25 mm above the liquid level of liquid nitrogen), vapor phase freezing (40 mm above the liquid level of liquid nitrogen) or vapor phase freezing (80 mm above the liquid level of liquid nitrogen) were thawed in Example 8 (8-1). In the metal contact method, a linear aggregate (fold) of dead cells has occurred.

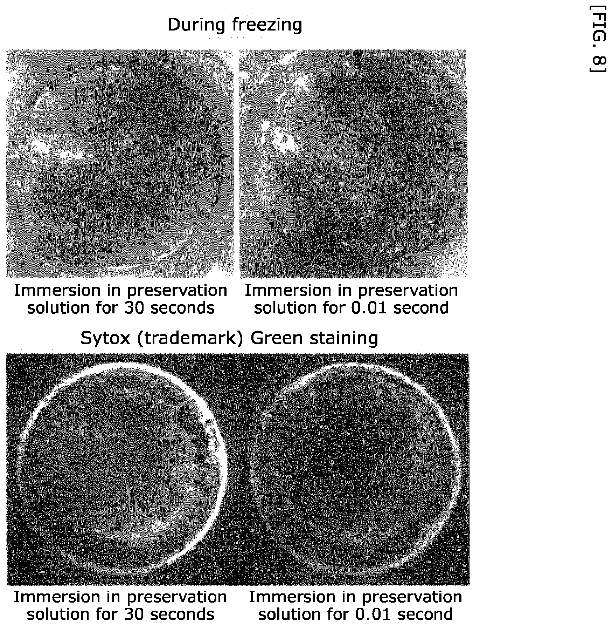

FIG. 8 shows photographs of vitrification freezing (upper) and photographs of SYTOX (trademark) Green staining after thawing (lower) when the immersion time in a preservation solution was 0.01 second or 30 seconds regarding a laminate of a thinned collagen gel and an RPE cell sheet in Example 8 (8-3).

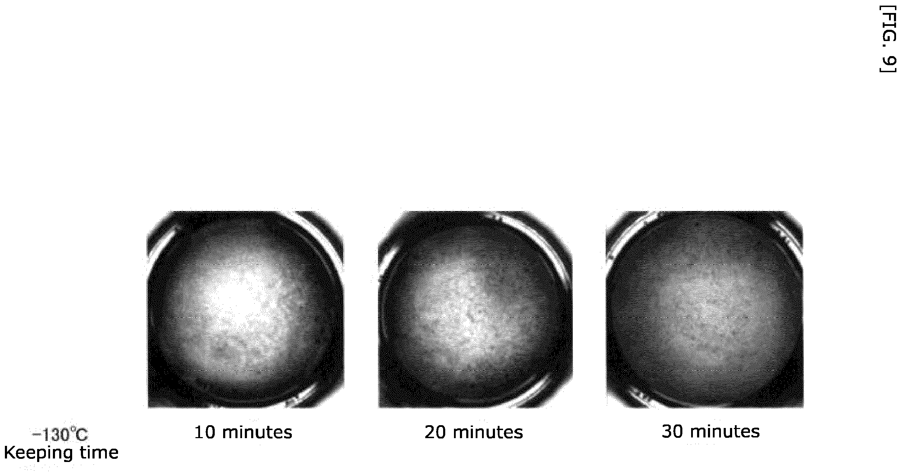

FIG. 9 shows photographs of an RPE cell sheet that was kept (10 minutes, 20 minutes and 30 minutes) at -130°C before immersing in a thawing solution in Example 9 (9-1).

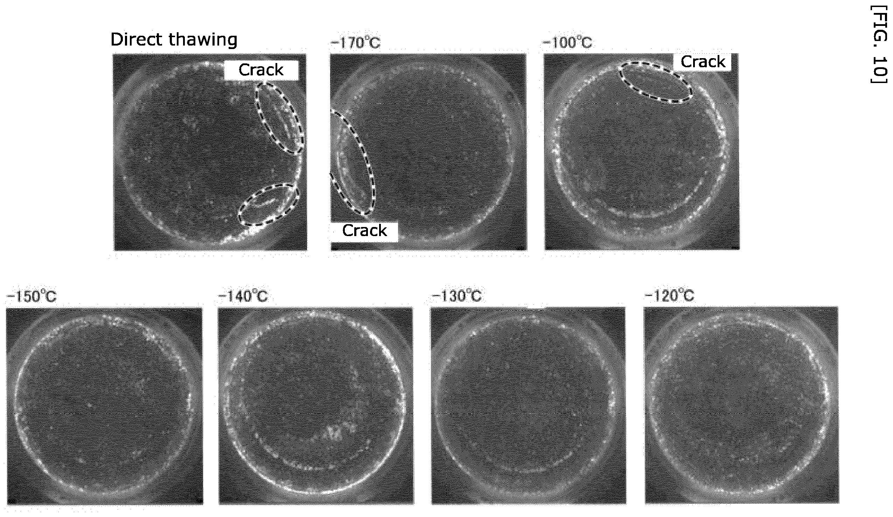

FIG. 10 shows photographs of SYTOX (trademark) Green staining after a laminate of a collagen gel and an RPE cell sheet was kept at a predetermined temperature (-170°C, -150°C, -140°C, -130°C, -120°C or -100°C) before immersing in a thawing solution and then thawed in Example 9 (9-2). At -170°C and -100°C, cracks have occurred.

### DETAILED DESCRIPTION OF THE INVENTION

According to one embodiment of the present disclosure, a method for producing a frozen body of a three-dimensional tissue aggregate of retinal pigment epithelial cells (hereinafter also referred to as "RPE") is characterized by including a step of preparing the three-dimensional tissue aggregate of the retinal pigment epithelial cells, and a step of subjecting the three-dimensional tissue aggregate of the retinal pigment epithelial cells to a freezing treatment at a predetermined freezing temperature set in advance using, as an index, the occurrence rate of a linear aggregate of dead cells in the three-dimensional tissue aggregate of the retinal pigment epithelial cells after thawing, thus obtaining the frozen body.

Details of the present disclosure will be described for each step.

### <Step of Preparing Three-Dimensional Tissue Aggregate of Retinal Pigment Epithelial Cells>

According to one embodiment of the present disclosure, a three-dimensional tissue aggregate of retinal pigment epithelial cells is prepared.

### <RPE Cells and Three-Dimensional Tissue Aggregate Thereof>

The retina in an eye includes photoreceptors that sense light at various levels, and interneurons that relay signals from the photoreceptors to retinal ganglion cells. These photoreceptors are cells having the highest metabolic activity in the eye, and metabolically and functionally supported by retinal pigment epithelial cells.

Due to many pathological conditions, patients' ability to grasp visual images may be deteriorated or completely lost (for example, trauma to eyes, infection, degeneration, vascular abnormality and inflammatory disease). The central part of the retina is known as macula, and is responsible for central vision, fine visualization and discrimination of colors. The functions of the macula may be adversely affected due to age-related macular degeneration (exudative type or atrophic type), diabetic macular edema, idiopathic choroidal neovascularization, high myopic macular degeneration or advanced retinitis pigmentosa, among the pathological conditions.

If age-related macular degeneration occurs, it often becomes the cause of vision loss in the center of the visual field (macula) due to retinal damage. Macular degeneration has been the major cause of visual impairment in the elderly (aged >50 years). The occurrence type of macular degeneration includes the "exudative" type and the "atrophic" type. In the atrophic type, cell debris (drusen) is/are accumulated between the retina and the choroid to press the retina, possibly resulting in, in some cases, retinal detachment and vision loss. In the case of a more serious exudative type, neovessels from the choroid exude into a space in the posterior macula, and as a result, photoreceptors and supporting cells thereof are killed. If functional cells are lost in the eye, neovessels become fragile, and leakage of blood and interstitial fluid frequently occurs, and as a result, further damage to the macula may occur.

RPE cells are one source of therapeutic cells for cell therapy of the late stage of atrophic age-related macular degeneration (AMD). The RPE cells can function as differentiated and polarized monolayers. When cryopreservation of differentiated and polarized RPE monolayers derived from stem cells is successful, there are benefits in the uniformity of clinical products, maximization of product yield, assurance of quality control in clinical product lots, and distribution of similar final products to remote clinical sites. In order to perform scale-up in accordance with current Good Manufacturing Practice (cGMP) and Good Tissue Practice (GTP), it is necessary to perform lot testing and final release testing that can be implemented by manufacturers. Low-temperature processes for freezing and thawing are currently used for adult cord blood, and autologous transplantation materials or allogeneic transplantation materials such as embryo-derived pluripotent stem cell lines and non-polarized embryonic stem cell-derived RPE (hESC-RPE), but according to the present disclosure, it is possible to provide a method that can be applied to a wide range of RPE cell sheets. Therefore, according to one embodiment of the present disclosure, the RPE cells are materials for autologous transplantation or allogeneic transplantation.

It is preferable that the RPE cells form a sheet-like cell group in terms of using in combination with a transplantation material for eyes. Therefore, according to a preferred embodiment of the present disclosure, the three-dimensional tissue aggregate of retinal pigment epithelial cells is an RPE cell sheet.

### <Cell Carrier>

The three-dimensional tissue aggregate of RPE cells may be a subject for a freezing treatment alone, but can be a subject for a freezing treatment together with structures other than the three-dimensional tissue aggregate (cell aggregate, tissue, scaffold, etc.) or a preservation solution or the like, as necessary. According to one embodiment of the present disclosure, the RPE cells or the three-dimensional tissue aggregate thereof are/is arranged on a carrier as a scaffold. A cell carrier is advantageous in that mechanical stability is imparted to cells and a high survival rate occurs after a thawing process.

As the carrier, it is possible to use a known biocompatible component as a cell carrier for culture and/or cryopreservation of cells or tissues. Examples of such biocompatible component include hyaluronic acid, alginate, agarose, fibrin, chitin/chitosan, polylactide (PLA), polyglycolide (PGA), poly-L-lactic acid (PPLA), gelatin or collagen or the like, but because of production of no toxic degradation product and biocompatibility, the biocompatible component is preferably hyaluronic acid, fibrin, gelatin or collagen, and more preferably collagen. Examples of the collagen include porcine type I collagen obtained from a porcine tendon, or bovine type I collagen obtained from a bovine hide or skin. The biocompatible component may be a mixture of type I collagen and type III collagen, or a mixture of type I collagen and/or type III collagen and elastin.

The content of the biocompatible component in the carrier is not particularly limited as long as the biocompatible component can be used as a scaffold for cryopreservation, and, for example, the content can be 0.1 to 90(w/v)%, preferably 0.1 to 80(w/v)%, and more preferably 0.1 to 50(w/v)% relative to the total mass of raw materials constituting the carrier.

An additive in the carrier other than the above-mentioned biocompatible components is not particularly limited as long as the additive can be used as a scaffold for cryopreservation, and examples thereof include polyols (glycerol, ethylene glycol, butenediol, propenediol, sorbitol, etc.), lipids (vegetable oil, etc.), water or the like.

The carrier is preferably layered in terms of retaining an RPE cell sheet. The carrier is usually a monolayer, and, for example, in the case of a collagen carrier, cells or tissues are directly seeded in a culture dish covered with a simple collagen gel monolayer. Therefore, according to a preferred embodiment of the present disclosure, a step of preparing the three-dimensional tissue aggregate of the RPE cells includes a step of seeding the retinal pigment epithelial cells on the carrier layer to culture them. Another arrangement that is widely used for cryopreservation of cells or tissues in a collagen-based hydrogel is a sandwich arrangement, and, for example, cells may be cryopreserved between two layers of collagen gels, and the present disclosure also includes such embodiment.

When the carrier is layered, the thickness of the carrier layer is preferably thinner in terms of mechanical strength and making a cryoprotectant permeate through the inside efficiently and achieving complete vitrification. The mean value of the thickness of the carrier layer is, for example, 200 µm or less, preferably 150 to 10 µm, more preferably 100 to 15 µm, and still more preferably 70 to 20 µm.

A method for forming the carrier layer is not particularly limited, but in terms of forming a thin carrier layer, it is preferable to combine a centrifugation method with a drying method (air drying, etc.). Therefore, according to one embodiment, the carrier layer is obtained by centrifuging a carrier raw material to make it layered, and drying it.

The condition of centrifugation when the carrier layer is formed is not particularly limited, but, for example, the condition is 0.5 to 4 hours at 100 to 4,000 × g, and preferably 1,000 to 2,500 × g. The temperature in centrifugation can be usually 1 to 40°C, and preferably room temperature (1 to 30°C).

The condition of drying after centrifugation is not particularly limited, but, for example, the condition can be usually 10 to 36 hours at 1 to 40°C, and preferably room temperature (1 to 30°C). Drying after centrifugation can be performed by, for example, allowing the carrier to stand in a clean bench.

### <Pretreatment Step>

According to one embodiment of the present disclosure, in terms of reducing the burden of a rapid change in osmotic pressure on cells, a pretreatment step in which the RPE cells or the three-dimensional tissue aggregate thereof are/is subjected to a dehydration treatment is performed. Performing the pretreatment step is also preferable in terms of preventing the destruction of the cell structure due to formation of ice crystals in freezing and thawing treatments. According to a preferred embodiment, the pretreatment step includes a step of bringing the RPE cells or the three-dimensional tissue aggregate thereof into contact with a dehydrating agent. Bringing the RPE cells or the three-dimensional tissue aggregate thereof into contact with a dehydrating agent is performed by immersing the RPE cells or the three-dimensional tissue aggregate thereof in a pretreatment solution containing a dehydrating agent. The RPE cells or the three-dimensional tissue aggregate thereof may be immersed in a pretreatment solution together with the carrier.

The dehydrating agent is not particularly limited as long as it has a dehydrating function, but the dehydrating agent is preferably polyhydric alcohol, more preferably polyethylene glycol, dextran, hydroxyethyl starch, polyvinyl alcohol, polyvinylpyrrolidone, Percoll or ethylene glycol, and still more preferably ethylene glycol. Polyhydric alcohol such as ethylene glycol can also be used as a cryoprotective agent in the step of immersing in a preservation solution mentioned below.

A solvent in the pretreatment solution is preferably a medium (a DMEM medium, a F12 HAM medium, a B27 medium, an EMEM medium, an RPMI1640 medium, an F12 medium, a TC199 medium, a GMEM medium, an αMEM medium, etc.), phosphate-buffered saline (PBS) or water or the like, and more preferably a medium. Here, the medium may contain amino acid or cell growth factor or the like.

The concentration of polyhydric alcohol in the pretreatment solution may be appropriately adjusted according to the state of cells, but the concentration is 1 to 30(w/v)%, more preferably 2 to 25(w/v)%, still more preferably 2 to 20(w/v)%, and yet more preferably 3 to 20(w/v)%.

The pretreatment solution may contain, in addition to a dehydrating agent, additives such as sugar and polyamino acid or a derivative thereof or the like. The specific type of sugar and polyamino acid or a derivative thereof is preferably the same as of the preservation solution mentioned below. The content of sugar and polyamino acid or a derivative thereof in the preservation solution is preferably lower than that of the preservation solution in terms of avoiding cytotoxicity, and the content is more preferably 1/128 to 1/2 relative to the content in the preservation solution mentioned below.

In the pretreatment step, the frequency of immersing cells in the pretreatment solution may be once or a plurality of times, but in terms of efficient dehydration, it is preferable to perform a plurality of times. More specifically, the frequency of immersing cells in the pretreatment solution is preferably three times or more, more preferably 3 to 8 times, and still more preferably 3 to 5 times.

In the pretreatment step, the total time during which the RPE cells or the three-dimensional tissue aggregate thereof are/is immersed in the pretreatment solution is, for example, 1 to 120 minutes, preferably 1 to 60 minutes, and more preferably 1 to 50 minutes. When the pretreatment step is performed a plurality of times, the time of each pretreatment step is, for example, 1 to 20 minutes, preferably 1 to 15 minutes, preferably 1 to 10 minutes.

The temperature of immersion in the pretreatment solution used in the pretreatment step is not particularly limited, but, for example, the temperature is usually 1 to 40°C, and preferably room temperature (1 to 30°C).

A specific immersion method in the pretreatment step is not particularly limited, but immersion can be performed by, for example, seeding RPE cells through a carrier on a membrane on the bottom of a commercially available Transwell (trademark) insert to form a three-dimensional tissue aggregate, and then pouring a pretreatment solution into the inside and the outside of the Transwell (trademark) insert so that the RPE cells or the three-dimensional tissue aggregate thereof are/is immersed in the pretreatment solution, followed by allowing to stand.

### <Step of Immersing in Preservation Solution (Treating with Vitrification Solution)>

After the above-mentioned pretreatment step, in terms of imparting freezing resistance, it is preferable that the RPE cells or the three-dimensional tissue aggregate thereof are/is immersed in a preservation solution (also referred to as "vitrification solution"). The RPE cells or the three-dimensional tissue aggregate thereof may be immersed in a preservation solution together with the carrier.

According to a preferred embodiment of the present disclosure, as the preservation solution, it is possible to use a known cryoprotective agent such as polyethylene glycol, dimethyl sulfoxide, glycerin, ethylene glycol, propylene glycol, propane diol or the like. Regarding the cryoprotective agent used as the preservation solution, one cryoprotective agent may be contained, or two or more cryoprotective agents may be contained. A preferable cryoprotective agent is ethylene glycol.

The concentration of the cryoprotective agent in the preservation solution is, for example, 1 to 60(w/v)%, preferably 3 to 50(w/v)%, and more preferably 5 to 40(w/v)%.

Examples of other components contained in the preservation solution of the present disclosure include saccharides such as sucrose, trehalose, glucose, raffinose, lactose, maltose, mannose, galactose and fructose, or the like. Preferably, the saccharide is sucrose. When the saccharide is contained in the preservation solution, one saccharide may be contained, or two or more saccharides may be contained.

The concentration of the saccharide in the preservation solution is, for example, 0.05 to 1.5 M, preferably 0.1 to 1.2 M, and more preferably 0.15 to 1.0 M.

From the viewpoint of improving the freezing resistance, polyamino acid including carboxylated polylysine (carboxylated ε-poly-L-lysine) mentioned in Patent Document 1 or a derivative thereof can also be preferably used as a component of the preservation solution. In the carboxylated polylysine, the ratio of the carboxyl group to the amino group (carboxyl group/amino group) can be, for example, in the range of 0.8 to 19, preferably in the range of 1.0 to 18, and more preferably in the range of 1.5 to 15.

The concentration of the polyamino acid or a derivative thereof in the preservation solution is, for example, 1 to 20(w/v)%, preferably 2 to 17(w/v)%, and more preferably 3 to 15(w/v)%.

Examples of the solvent in the preservation solution include a medium (a DMEM medium, an F12 HAM medium, a B27 medium, an EMEM medium, an RPMI1640 medium, an F12 medium, a TC199 medium, a GMEM medium, an αMEM medium, etc.) or physiological saline (PBS, etc.), but the solvent is preferably a medium.

According to a preferred embodiment of the present disclosure, the preservation solution contains ethylene glycol, sucrose, polyamino acid or a derivative thereof (preferably carboxylated polylysine).

The time during which the RPE cells are immersed in the preservation solution (time during which the cells are brought into contact with the preservation solution) depends on the composition of the vitrification solution used, but in many cases, in terms of avoiding the chemical toxicity of the preservation solution, the time is preferably within 60 seconds. The time is more preferably 0.01 to 60 seconds, and still more preferably 0.01 to 30 seconds.

### <Step of Obtaining Frozen Body>

According to one embodiment of the present disclosure, the three-dimensional tissue aggregate of RPE cells subjected to the dehydration treatment and the treatment of immersing in a preservation solution mentioned above is subjected to a freezing treatment. According to a preferred embodiment of the present disclosure, the freezing condition including the freezing temperature at which the three-dimensional tissue aggregate of RPE cells is subjected to a freezing treatment is set in advance using, as an index, the occurrence rate of a linear aggregate of dead cells in the three-dimensional tissue aggregate of the RPE cells after thawing.

It is a surprising fact that a linear aggregate (hereinafter also referred to as "fold") of dead cells may appear in the three-dimensional tissue aggregate of the RPE cells after thawing, which affects the quality or the cryopreservation resistance as a transplantation material including a transepithelial electric resistance value (TER value).

The freezing condition can be set by the following procedures. Such procedures are also used in Examples mentioned below. 1) Prepare a plurality of test samples of a three-dimensional tissue aggregate of RPE cells obtained under the same freezing and thawing conditions although the freezing condition is different. Here, the freezing condition is not particularly limited, and examples thereof include freezing temperature, temperature fall rate, freezing method or the like, but the freezing condition is preferably freezing temperature.

2) The three-dimensional tissue aggregate of the RPE cells thus obtained is fluorescently stained with SYTOX (trademark) Green.

3) The three-dimensional tissue aggregate of the RPE cells that was fluorescently stained is used as a measurement sample, and the measurement sample is excited through a microscope excitation filter at a wavelength of 470 to 490 nm, and is fluorescently detected through a microscope absorption filter at a wavelength of 510 to 550 nm.

4) Fluorescence is photographed with a CCD camera mounted on a fluorescence microscope. When the three-dimensional tissue aggregate of the RPE cells is sheet-like, photographs should be taken from one side of the sheet. The magnification of the microscope is 4 to 10 times for the objective lens and 10 times for the ocular lens. In photographing, images can be obtained by using image analysis software (OLYMPUS cellSens Demension: exposure time of 200 ms, resolution (number of pixels) of 1360 × 1024).

Here, "linear aggregate of dead cells" means an aggregate of three or more dead cells that are linearly arranged in which the distance between dead cells that are adjacent to each other is less than 10 µm. The adjacent dead cells may be separated or in contact with each other as long as the distance is within the range of less than 10 µm, but preferably the dead cells are in contact with each other. "Linear" includes not only linear but also curved and a combination thereof (a combination of a straight line and a straight line, a combination of a straight line and a curve, and a combination of a curve and a curve).

The dead cells and the linear aggregate thereof can be detected using the above-mentioned fluorescence as an index, and the distance between adjacent dead cells can be determined by measuring the distance between respective center points of fluorescence on images obtained by photographing using the above-mentioned image analysis software. Whether adjacent dead cells are in contact with each other or not can be determined using the overlap of fluorescence corresponding to dead cells as an index.

5) The occurrence rate of a linear aggregate of dead cells in the three-dimensional tissue aggregate of the retinal pigment epithelial cells after thawing is preferably set in advance using, as an index, the total length of linear aggregates existing in the three-dimensional tissue aggregate. Specifically, after measurement of the total length of linear aggregates recognized using fluorescence as an index on a photographed image of the three-dimensional tissue aggregate of the RPE cells, it is possible to select a freezing condition in which the total length of linear aggregates is, for example, 100 µm or less, preferably 50 µm or less, and more preferably 30 µm or less.

It is still more preferable that the freezing condition is set using, as an index, the fact that no fold has occurred after thawing. Therefore, according to a preferred embodiment of the present disclosure, the above-mentioned freezing treatment step includes a step of adjusting the freezing temperature to a predetermined range so that no linear aggregate of dead cells occurs in the frozen body of the three-dimensional tissue aggregate after thawing. Such adjustment of the freezing condition is advantageous in efficiently producing a three-dimensional tissue aggregate of RPE cells of high quality.

The freezing temperature of the three-dimensional tissue aggregate of the RPE cells is usually -191°C to -105°C, preferably -185°C to -105°C, more preferably -185°C to -110°C, and still more preferably -180°C to -130°C, in terms of avoiding damage to the cells due to a rapid volume change. The above-mentioned freezing temperature is advantageous in efficiently producing a three-dimensional tissue aggregate of RPE cells of high quality.

According to one embodiment of the present disclosure, the freezing temperature of the three-dimensional tissue aggregate of the RPE cells is preferably the glass transition temperature of water or a vicinity region thereof, and more preferably -140°C ±40°C. Without being bound by any theory, freezing at near the glass transition temperature of water is considered preferable particularly in avoiding damage to the cells due to a volume change of water.

The temperature fall rate in the above-mentioned freezing treatment is, for example, more than 1°C/second, preferably in the range of 1.5°C/second to 144°C/second, and more preferably 1.5°C/second to 45°C/second.

According to one embodiment of the present disclosure, the above-mentioned freezing treatment is a vitrification freezing method. Regarding complete vitrification of the three-dimensional tissue aggregate of the RPE cells, freezing by complete vitrification is preferable in order to maintain the structure or morphology equivalent to that before freezing even after thawing.

Examples of a cooling agent used in the freezing treatment include liquid nitrogen, slush nitrogen or liquefied ethane or the like, but the cooling agent is preferably liquid nitrogen.

The freezing treatment is performed preferably in a vapor phase using a vaporized cooling agent. When the freezing treatment is performed in a vapor phase of a cooling agent, the freezing temperature may be adjusted by adjusting the distance from the liquid level of the cooling agent to the cells. The distance to the liquid level is, for example, 0 to 70 mm, and may be preferably 5 to 70 mm, and more preferably 5 to 40 mm. Here, when a three-dimensional tissue aggregate of RPE cells is frozen by vitrification in a vapor phase together with a carrier as a scaffold, the three-dimensional tissue aggregate of the RPE cells may be near the liquid level of the cooling agent or the carrier may be near the liquid level of the cooling agent.

By the above-mentioned method, it is possible to produce a frozen body of a three-dimensional tissue aggregate of RPE cells.

### <Storage and Transportation>

According to the present disclosure, a three-dimensional tissue aggregate of RPE cells is made into a frozen body, and the frozen body can be stably preserved or transported in a freezing environment. Here, examples of the freezing environment include a storage cabinet (freezer, etc.) or means of transportation (vehicle, aircraft, etc.) equipped with refrigeration equipment at the temperature at which the frozen body of the three-dimensional tissue aggregate of the RPE cells is stored in a frozen state. The temperature at which the frozen body is stored in a frozen state is, for example, about -150°C to -196°C.

According to one embodiment of the present disclosure, provided is a method for preserving a three-dimensional tissue aggregate of RPE cells, the method including a step of preserving a frozen body of the three-dimensional tissue aggregate of the RPE cells in a cryopreservation environment. According to another embodiment, provided is a method for transporting a three-dimensional tissue aggregate of RPE cells, the method including a step of transporting a frozen body of the three-dimensional tissue aggregate of the RPE cells in a cryopreservation environment.

According to another embodiment of the present disclosure, the frozen body of the three-dimensional tissue aggregate of the RPE cells may be transported after being subjected to a thawing treatment according to the method mentioned below. Therefore, according to a preferred embodiment of the present disclosure, provided is a method for transporting a three-dimensional tissue aggregate of RPE cells, the method including a step of transporting a thawed product obtained by thawing the above-mentioned frozen body of the three-dimensional tissue aggregate of the RPE cells. The transport temperature of the thawed product may be a low temperature or ordinary temperature, and more specifically, the temperature may be, for example, in the range of 0 to 38°C, and preferably in the range of 10 to 38°C.

### <Thawing Treatment>

The frozen body of the three-dimensional tissue aggregate of the RPE cells is subjected to a thawing treatment when used.

According to a preferred embodiment of the present disclosure, in the thawing treatment, it is preferable to keep the frozen body within a predetermined temperature range in terms of suppressing a rapid volume change. Such thawing treatment is advantageous in avoiding the occurrence of cracks in the three-dimensional tissue aggregate of the RPE cells during thawing.

The temperature at which the three-dimensional tissue aggregate of the RPE cells is kept during thawing is usually -195 to -105°C, preferably -165°C to -105°C, more preferably -160°C to -110°C, and still more preferably -155°C to -115°C, in terms of avoiding the occurrence of cracks during thawing.

According to one embodiment of the present disclosure, the temperature at which the three-dimensional tissue aggregate of the RPE cells is kept during thawing is preferably the glass transition temperature of water or a vicinity region thereof, and more preferably -140°C ±40°C. Without being bound by any theory, thawing at near the glass transition temperature of water is, like freezing, considered preferable particularly in avoiding damage to the cells due to a volume change of water.

The time during which the three-dimensional tissue aggregate of the RPE cells is kept during thawing is, for example, 1 to 60 minutes, preferably 1 to 50 minutes, and more preferably 5 to 40 minutes.

The step of keeping the three-dimensional tissue aggregate of the RPE cells during thawing may be performed, for example, like the freezing treatment, in a vapor phase using the same cooling agent as in the freezing treatment such as liquid nitrogen. Adjustment of the cooling temperature can be performed by, for example, referring to the distance between the cooling agent and the three-dimensional tissue aggregate and the temperature according to the method mentioned in Example 10.

### <Thawing Solution>

According to one embodiment of the present disclosure, the thawing treatment can be performed by adding a thawing solution to the frozen body. Addition of a thawing solution can be performed before and after or simultaneously with the step of keeping the three-dimensional tissue aggregate of the RPE cells within a predetermined temperature range during thawing, but the addition is preferably performed after the step of keeping within a predetermined temperature range mentioned above.

As the thawing solution added to the frozen body, it is possible to use a thawing solution containing sugar or sugar alcohol. In a suitable embodiment, the thawing solution is a medium containing sugar or sugar alcohol. As the sugar or sugar alcohol, for example, it is possible to use sugar or sugar alcohol selected from the group consisting of sucrose, trehalose, glucose, raffinose, maltose, fructose, inulin and fructan, but sucrose is preferable.

As the concentration of the sugar or sugar alcohol, for example, it is possible to use a concentration in the range of 0.1 to 2 M, preferably 0.2 to 1.5 M, and more preferably 0.5 to 1.2 M. Usually, as the thawing solution, a solution containing a medium or a component thereof and containing sugar or sugar alcohol is used. As the medium, it is possible to use usual media used for RPE cells, and these are as mentioned above regarding the pretreatment solution and the preservation solution.

### <Addition of Thawing Solution>

The thawing solution can be directly added to the frozen body. In a suitable embodiment, a thawing solution warmed to a temperature, for example, in the range of 20 to 38°C, and preferably in the range of 30 to 38°C can be added at an ambient temperature, for example, in the range of 20 to 38°C, and preferably in the range of 30 to 38°C to perform thawing.

### <Washing>

In a suitable embodiment, to the three-dimensional tissue aggregate of the RPE cells that was thawed by adding the thawing solution, after disposal of the thawing solution, it is possible to further add a culture medium (maintenance medium) to wash the cells. In a suitable embodiment, a culture medium warmed to a temperature, for example, in the range of 20 to 38°C, and preferably in the range of 30 to 38°C can be added at an ambient temperature, for example, in the above-mentioned range of 20 to 38°C, and preferably in the range of 30 to 38°C. As the medium, it is possible to use usual media used for RPE cells, and these are as mentioned above regarding the pretreatment solution and the preservation solution.

According to one embodiment of the present disclosure, provided is a frozen body of a three-dimensional tissue aggregate of RPE cells or a thawed product thereof obtained by the above-mentioned method.

The area of the three-dimensional tissue aggregate of the RPE cells and the frozen body thereof is not particularly limited, and may be determined by considering the size used as a graft, and, for example, the area is 0.001 to 50 cm², and preferably 0.01 to 5 cm².

It is preferable that the TER value before freezing of the three-dimensional tissue aggregate of the RPE cells is substantially equivalent to the TER value after freezing, and that there is no significant difference. The TER value of the three-dimensional tissue aggregate of the RPE cells is, for example, 70 to 1,000, preferably 130 to 900, and more preferably 150 to 850.

The frozen body or the three-dimensional tissue aggregate obtained by thawing it can be used as a pharmaceutical in the treatment of age-related macular degeneration and the like. Therefore, according to one embodiment, provided is a pharmaceutical composition including a frozen body of a three-dimensional tissue aggregate of RPE cells or a thawed product thereof. According to another embodiment, the above-mentioned frozen body, thawed product and pharmaceutical composition are used for regenerative medicine. According to a preferred embodiment, the above-mentioned frozen body, thawed product and pharmaceutical composition are materials for transplantation, and preferably cell sheets.

According to a preferred embodiment, the above-mentioned frozen body, thawed product and pharmaceutical composition are used for allogeneic transplantation. The above-mentioned frozen body of the present disclosure can be advantageously used in allogeneic transplantation requiring collection from a donor in advance and storage since the three-dimensional tissue aggregate of the RPE cells can be stored while stably maintaining high quality for a long period of time.

According to one embodiment of the present disclosure, it is possible to provide the followings.
(1) A method for producing a frozen body of a three-dimensional tissue aggregate of retinal pigment epithelial cells, the method including:
   a step of preparing the three-dimensional tissue aggregate of the retinal pigment epithelial cells, and
   a step of subjecting the three-dimensional tissue aggregate of the retinal pigment epithelial cells to a freezing treatment at a predetermined freezing temperature set in advance using, as an index, the occurrence rate of a linear aggregate of dead cells in the three-dimensional tissue aggregate of the retinal pigment epithelial cells after thawing, thus obtaining the above-mentioned frozen body.
(2) The method according to (1), wherein the above-mentioned freezing temperature is -191°C to -105°C.
(3) The method according to (1) or (2), wherein the above-mentioned freezing treatment is performed in a vapor phase.
(4) The method according to (3), wherein the above-mentioned vapor phase includes liquid nitrogen gas.
(5) The method according to any one of (1) to (4), wherein the above-mentioned freezing treatment is performed by a vitrification freezing method.
(6) The method according to any one of (1) to (5), the method including a step of adjusting a freezing temperature of the three-dimensional tissue aggregate of the retinal pigment epithelial cells using, as an index, the occurrence rate of a linear aggregate of dead cells in the three-dimensional tissue aggregate of the retinal pigment epithelial cells after thawing.
(7) The method according to any one of (1) to (6), wherein the temperature fall rate in the above-mentioned freezing treatment is 1.5°C/second to 144°C/second.
(8) The method according to any one of (1) to (7), wherein the above-mentioned step of preparing the three-dimensional tissue aggregate includes a step of subjecting the above-mentioned retinal pigment epithelial cells to a dehydration treatment.
(9) The method according to (8), wherein the above-mentioned dehydration treatment includes a step of bringing the retinal pigment epithelial cells into contact with a dehydrating agent.
(10) The method according to (8) or (9), wherein the above-mentioned dehydration treatment is performed a plurality of times.
(11) The method according to (9) or (10), wherein the above-mentioned dehydrating agent includes polyhydric alcohol.
(12) The method according to any one of (9) to (11), which increases the concentration of the above-mentioned dehydrating agent in each dehydration treatment over time.
(13) The method according to (11) or (12), wherein the above-mentioned polyhydric alcohol includes ethylene glycol.
(14) The method according to any one of (1) to (13), wherein the above-mentioned step of preparing the three-dimensional tissue aggregate includes a step of immersing the above-mentioned retinal pigment epithelial cells in a preservation solution after the dehydration treatment.
(15) The method according to (14), wherein the above-mentioned immersion time is 0.01 to 60 seconds.
(16) The method according to (14) or (15), wherein the above-mentioned preservation solution includes at least a cryoprotective agent.
(17) The method according to any one of (1) to (16), wherein the above-mentioned three-dimensional tissue aggregate is arranged on a carrier layer.
(18) The method according to any one of (1) to (17), wherein the above-mentioned step of preparing the three-dimensional tissue aggregate includes a step of seeding the above-mentioned retinal pigment epithelial cells on the carrier layer to culture them.
(19) The method according to (17) or (18), wherein the above-mentioned carrier layer includes collagen as a principal component.
(20) The method according to any one of (17) to (19), wherein the mean thickness of the above-mentioned carrier layer is 200 µm or less.
(21) The method according to any one of (1) to (20), wherein the above-mentioned three-dimensional tissue aggregate of the retinal pigment epithelial cells is a sheet of the above-mentioned retinal pigment epithelial cells.
(22) The method according to (21), wherein the above-mentioned sheet of the retinal pigment epithelial cells is a monolayer.
(23) A frozen body of a three-dimensional tissue aggregate of retinal pigment epithelial cells, which is obtained by the method according to any one of (1) to (22).
(24) A method for preserving a three-dimensional tissue aggregate of retinal pigment epithelial cells, the method including a step of preserving the frozen body according to (23) in a cryopreservation environment.
(25) A method for transporting a three-dimensional tissue aggregate of retinal pigment epithelial cells, the method including a step of transporting the frozen body according to (23) in a cryopreservation environment.
(26) A method for producing a three-dimensional tissue aggregate of retinal pigment epithelial cells, the method including a step of thawing the frozen body according to (23).
(27) The method according to (26), wherein the above-mentioned step of thawing the frozen body includes a step of keeping the above-mentioned frozen body at near the glass transition temperature of water.
(28) The method according to (26) or (27), wherein the above-mentioned step of thawing the frozen body includes a step of keeping the above-mentioned frozen body at -160°C to -110°C.
(29) The method according to (27) or (28), wherein the time to keep the above-mentioned frozen body is 1 to 60 minutes.
(30) The method according to any one of (26) to (29), wherein the above-mentioned step of thawing the frozen body includes a step of keeping the above-mentioned frozen body in a thawing solution.
(31) A method for transporting a three-dimensional tissue aggregate of retinal pigment epithelial cells, the method including a step of transporting the thawed product obtained in the step according to (26).

### EXAMPLES

Examples of the present disclosure will be described below, but the present disclosure is not limited to these Examples.

Cryopreservation and use of RPE cells can be broadly divided into four steps of pretreatment (dehydration), immersion in a preservation solution, freezing and thawing.

### Example 1

### Investigation of Pretreatment Solution (Dehydration Solution)

In order to investigate the effects of the concentration of ethylene glycol (EG) and a diluting solvent in a pretreatment solution for freezing of RPE cells, toxicity evaluation was performed in accordance with the following procedures.

RPE cells (manufactured by Lonza K.K., 00194987) were seeded on a 96-well plate (Falcon, 353072) at 3.3 × 10⁴ cells/well, and cultured in a maintenance medium (350 mL of DMEM (Sigma, D6046), 150 mL of F12 HAM (Sigma, N6658), 10 mL of B27 (Invitrogen, 17504-044), 5 mL of 200 mM L-glutamine (Sigma, G7513), 10ng/mL of bFGF (Wako, 060-04543), 0.5 mM of SB431542 hydrate (Sigma, S4317)) until the cells reached confluent. The cultured cells were washed with PBS(-) at room temperature, and then exposed to a pretreatment solution for 10 minutes or 30 minutes. Then, the cultured cells were washed with the maintenance medium twice, followed by treatment for 1.5 hours using tetrazolium salt (MTS:
[3-(4,5-dimethylthiasol-2-yl)-5-(3carboxymethoxyphenyl-2-(4-sulf ophenyl)-2H-tetraxolium, inner salt]), and the metabolic activity of the cells at 0 hours of culture after exposure was confirmed by absorbance measurement (MTS assay). Also in the other wells, the same pretreatment and washing with PBS(-) as mentioned above were performed, and the cultured cells were continuously cultured in the maintenance medium, followed by an MTS assay at a designated time.

As the pretreatment solutions, solutions having EG concentrations of 10(v/v)%, 12.5(v/v)%, 15(v/v)%, 17.5(v/v)% and 20(v/v)% were used. As the solvent of the pretreatment solution, a Dulbecco's modified Eagle's medium (DMEM) supplemented with 2 mM L-glutamine or PBS(-) was used.

Results when the DMEM medium supplemented with 2 mM L-glutamine (Sigma, G7513) was used as the solvent and an exposure time was 10 minutes are as shown in FIG. 1.

In FIG. 1, the initial toxicity at 0 hours of culture time is increased in a concentration-dependent manner from the EG concentration of 12.5(v/v)%. Particularly, a great difference was observed in the initial toxicity between the EG concentrations of 12.5(v/v)% and 15(v/v)%.

It was confirmed that recovery is possible in about 24 hours of culture time at up to the EG concentration of 17.5(v/v)%.

Even at 30 minutes of exposure time to the pretreatment solution, the same tendency of cytotoxicity as in the results of FIG. 1 was confirmed. However, a tendency in which a longer time to recovery is required in 30-minute exposure was confirmed.

Even when PBS(-) was used as the solvent, the same tendency as when the DMEM medium supplemented with glutamine was used was observed. However, a tendency in which the cell metabolic activity is higher in the DMEM medium supplemented with glutamine was confirmed.

### Example 2

From the results of Example 1, since cytotoxicity is suppressed to the minimum and a high dehydration effect is expected, a pretreatment method using a pretreatment solution (DMEM medium supplemented with glutamine) having an EG concentration of 15(v/v)% such that the RPE cells can maintain the sheet structure even after the thawing step was investigated. Specifically, a stepwise treatment method in which a diluted solution of the pretreatment solution is used stepwise a plurality of times was compared with a single treatment method in which the pretreatment solution is used once.

### Preparation of Test Sample

### (Preparation of Collagen Gel Mixed Solution)

A: 3.0 mg/mL of Cellmatrix Type I-A (porcine tendon-derived acid-solubilized Type I collagen, Nitta Gelatin Inc.), B: a 5-fold concentrated culture solution (a solution obtained by dissolving 3 g of DMEM/F12 (Invitrogen, 12500-062) in MilliQ water, and subjecting a total volume of 50 mL to a filter treatment), and C: a buffer for reconstitution (Nitta Gelatin Inc.) were prepared. While cooling, B (2 volumes) was added to A (7 volumes), and they were mixed without causing foaming. Then, C (1 volume) was added to the mixed solution thus obtained and mix them to obtain a 0.21(w/v)% collagen gel mixed solution.

### (Fabrication of RPE Cell Sheet)

Into a Transwell (trademark) (Corning, #3460) insert, 150 µL of the 0.21(w/v)% collagen gel mixed solution was added dropwise, followed by incubation at 37°C for 30 minutes. Then, 500 µL of an F-10 medium (F-10 HAM (Sigma, N6908)) containing 10(v/v)% FBS and 1(v/v)% Penicillin-Streptomycin (Invitrogen, 15140-122) was added to the inside of the insert, and 1,500 µL of the same was added to the outside of the insert, followed by incubation at 37°C for 24 hours. The next day, the inside and the outside of the insert were washed once with an F-10 medium supplemented with 10(v/v)% FBS, and RPE cells (manufactured by Lonza K.K., 00194987) were seeded into the inside of the insert so that the number of cells were 5.0 × 10⁵ (F-10 medium supplemented with 10(v/v)% FBS, 500 µL), and 1,500 µL of the F-10 medium supplemented with 10(v/v)% FBS was added to the outside of the insert. Until the cells reached confluent, culture was performed in the F-10 medium supplemented with 10(v/v)% FBS, and when the cells reached confluent, the medium was replaced with the above-mentioned maintenance medium, and culture was continued. Medium replacement was performed twice to three times/week.

### Stepwise Treatment Method

In accordance with the above-mentioned procedures, an RPE cell sheet was fabricated in the Transwell (trademark) (Corning, #3460) insert. Then, five pretreatment solutions having different concentrations (EG concentrations of 3, 6, 9, 12 and 15(v/v)%) were prepared by 5-serial dilution. Then, 500 uL of a 3(v/v)% pretreatment solution was added to the inside of the Transwell (trademark) insert, and 1,000 uL of the same was added to the outside of the insert. By allowing to stand at room temperature for 2 minutes, a dehydration treatment was performed, followed by replacement with a pretreatment solution having an EG concentration of 6(v/v)%. Furthermore, replacement of pretreatment solutions was repeated by the same procedures. Pretreatment solutions were used in the concentration order from 3(v/v)% to 15(v/v)%, and a stepwise dehydration treatment for a total of 2 minutes × 5 times was performed.

### Single Treatment Method

In accordance with the above-mentioned procedures, an RPE cell sheet was fabricated in the Transwell (trademark) (Corning, #3460) insert. Then, 500 uL of a pretreatment solution (EG concentration of 5, 10, 15 or 20(v/v)%) was added to the inside of the Transwell (trademark) insert and 1,000 uL of the same was added to the outside of the insert. By allowing to stand at room temperature for 10 minutes, a dehydration treatment was performed.

### Immersion in Preservation Solution, Freezing and Thawing

In either case of the stepwise treatment method or the single treatment method, the following immersion in a preservation solution, freezing and thawing were performed in the same manner. Specifically, the pretreatment solution was replaced with a preservation solution StemCell Keep (Bioverde) (0°C) of the same volume, and the preservation solution was removed as possible, followed by allowing to stand for 5 minutes while cooling in ice.

Then, the preservation solution was removed, and the RPE cell sheet was frozen in a vapor phase of liquid nitrogen, followed by allowing to stand for 10 minutes.

Then, the frozen RPE cell sheet was immersed in a 1 M sucrose (Suc) solution at 37°C together with Transwell (trademark) and an insert thereof, and thawed. After confirmation of the thawed state, the RPE cell sheet was transferred to a 12-well plate and allowed to stand. At the time point when 1 minute elapsed from thawing, the preexisting Suc solution was disposed of, and 500 uL of a 0.5 M Suc solution at room temperature was added to the inside of the insert and 1,000 uL of the same was added to the outside of the insert, followed by allowing to stand for 3 minutes. Then, replacement with a maintenance medium of the same volume at room temperature was performed, followed by allowing to stand at room temperature for 5 minutes. Then, the medium was replaced with a fresh maintenance medium at room temperature, followed by allowing to stand at room temperature for 5 minutes. Then, the medium was replaced with a fresh maintenance medium at room temperature, and the RPE cell sheet was incubated at 37°C.

Thereafter, culture was performed in the maintenance medium for 3, 7 or 14 days. In the RPE cell sheet on day 3 of culture, SYTOX (trademark) Green was added to the inside of the Transwell (trademark) insert, followed by incubation at 37°C, and dead cells were fluorescently stained.

As a result, observation of cell morphology (observation magnification of ×100 and x200) with a microscope (manufactured by Olympus Corporation, IX83) was as shown in FIG. 2.

FIG. 2 shows photographs of untreated (pretreatment, freezing and thawing are not performed) RPE cell sheets, photographs of RPE cell sheets obtained by the single treatment method (pretreatment for 10 minutes × once; EG concentration of 5, 10, 15 or 20(v/v)%), and photographs of RPE cell sheets obtained by the stepwise treatment method (pretreatment for 2 minutes × 5 times; EG concentrations of 3, 6, 9, 12 and 15(v/v)%). When compared with the single treatment method, the cell morphology was more maintained in the stepwise treatment method at any of the time points of days 3, 7 and 14 of culture.

FIG. 3 shows photographs of RPE cell sheets stained using SYTOX (trademark) Green on day 3 of culture (observation magnification of ×100). The upper photographs are fluorescent staining photographs of RPE cell sheets obtained by the single treatment method (pretreatment for 10 minutes × once; EG concentration of 5, 10, 15 or 20(v/v)%), the middle photograph is a fluorescent staining photograph of an RPE cell sheet obtained by the stepwise treatment method (pretreatment for 2 minutes × 5 times; EG concentrations of 3, 6, 9, 12 and 15(v/v)%), and the lower photograph is a fluorescent staining photograph of an untreated RPE cell sheet. Regarding the detection of dead cells by SYTOX (trademark) Green staining, the number of dead cells was lower in the RPE cell sheet in the stepwise treatment method than that in the RPE cell sheets in the single treatment method. This is considered because, by using the stepwise treatment, a rapid change in osmotic pressure on RPE cells was suppressed, and occurrence of dead cells and deformation of cell morphology were prevented.

### Example 3

### 3-1

The effects when Suc and a cryoprotectant (carboxylated polylysine; COOH-PLL) are added to a pretreatment solution were investigated. Six groups shown below were prepared. As a stock solution of the pretreatment solution containing EG, Suc and COOH-PLL, StemCell Keep (trademark) (Bioverde) was used.

- Untreated group (no pretreatment solution)
- Control group (only DMEM medium supplemented with glutamine)
- EG 10% group (pretreatment solution having an EG concentration of 10(v/v)%)
- EG 20% group (pretreatment solution having an EG concentration of 20(v/v)%)

- EG 10% + Suc + COOH-PLL group (prepared by diluting a pretreatment solution (StemCell Keep (trademark) (Bioverde)) having an EG concentration of about 10(w/v)%, a Suc concentration of 0.188 M and a COOH-PLL concentration of 2.5(w/v)%)
- EG 20% + Suc + COOH-PLL group (prepared by diluting a pretreatment solution (StemCell Keep (trademark) (Bioverde)) having an EG concentration of about 20(w/v)%, a Suc concentration of 0.375 M and a COOH-PLL concentration of 5.0(w/v)%)

Using the above-mentioned six groups, toxicity evaluation (MTS assay) was performed in accordance with the description in Example 1.

As a result, it was confirmed that a decrease in cell metabolism is more suppressed in the EG 10% + Suc + COOH-PLL group and the EG 20% + Suc + COOH-PLL group compared with the EG10% group and the EG 20% group.

Regardless of containing an EG concentration of 20(w/v)%, the EG 20% + Suc + COOH-PLL group showed cytotoxicity almost equivalent to that in the EG 10% group.

From the test results, it is considered that, even at an equivalent EG concentration, concomitant use of Suc and COOH-PLL is suitable for maintaining the cell morphology. In the subsequent experiments, a pretreatment solution containing an EG concentration of 20(w/v)%, a Suc concentration of 0.375 M and a COOH-PLL concentration of 5(w/v)%, which is expected to have an effective dehydrating effect and an effect of relieving cytotoxicity, was to be used for the fifth step of the serial dilution method.

### Example 4

After the pretreatment step (dehydration step), in order to select a preservation solution used for immersing RPE cells (manufactured by Lonza K.K.) before freezing, a toxicity test (MTS assay) was performed in accordance with the procedures mentioned below.

### <Preservation Solution>

As the slow cryopreservation solution or the rapid cryopreservation solution, the following reagents were selected. Slow cryopreservation solution: STEM-CELLBANKER (trademark) (ZENOAQ)

Rapid cryopreservation solution: StemCell Keep (trademark) (Bioverde) (containing EG, Suc and COOH-PLL)

### <Exposure Conditions>

### As the exposure conditions, the followings were selected. Slow Cryopreservation Solution

(4-i) No exposure (= medium replacement once)
(4-ii) Medium at 37°C
(4-iii) Medium at 4°C
(4-iv) STEM-CELLBANKER (trademark) (4°C) - exposure time of 1 minute
(4-v) STEM-CELLBANKER (trademark) (4°C) - exposure time of 10 minutes
(4-vi) STEM-CELLBANKER (trademark) (4°C) - exposure time of 60 minutes

### Rapid Cryopreservation Solution

(4'-i) No exposure (= medium replacement once)
(4'-ii) Medium at 37°C
(4'-iii) Medium at 0°C
(4'-iv) Freezing Medium (trademark) (0°C) - exposure time of 20 seconds
(4'-v) StemCell Keep (trademark) (0°C) - exposure time of 20 seconds
(4'-vi) PBS(-) (0°C) - exposure time of 20 seconds (4'-vii) Freezing Medium (trademark) (0°C) - exposure time of 1 minute
(4'-viii) StemCell Keep (trademark) (0°C) - exposure time of 1 minute

In accordance with the same procedures as in Example 1, the metabolic activity of RPE cells was confirmed by absorbance measurement (MTS assay). As a result, in all preservation solutions, the shorter the immersion time was, the lower the toxicity was. The tendency of the severity of the toxicity was as shown below.

### (4-iv) < (4-v) < (4'-v), (4'-iv) ≤ (4'-viii) < (4-vi) < (4'-vii)

When the types of the preservation solutions were compared, a tendency in which CELLBANKER (trademark) had the lowest toxicity, followed by StemCell Keep (trademark) and Freezing Medium (trademark) in the order of lower to higher toxicity was observed.

As long as the time is within the recommended time of each cryopreservation solution (time to addition to cells, suspension and freezing, when used for the purpose of freezing), RPE cells are severely damaged, and the metabolism recovers within 24 hours.

Although the toxicity of the slow freezing solution CELLBANKER (trademark) is considered low, a tendency for the metabolic activity 48 hours after exposure to decrease for any exposure time, and thus there may be a possibility that the metabolism after recovery is unlikely to be stable. Thus, for freezing of RPE cells, the rapid freezing method in which the metabolic activity after exposure to a preservation solution is stable and which is considered to be suitable for freezing of a 3D tissue was to be selected.

### Example 5

In order to evaluate the damage after thawing, a thawing test on a suspension of RPE cells (manufactured by Lonza K.K.) was performed.

The following two preservation solutions were used, and conditions for immersion in a preservation solution and thawing were as follows.

### <Rapid Cryopreservation Solution>

StemCell Keep (trademark) (Bioverde) (containing EG, Suc and COOH-PLL) and Freezing Medium (trademark) for ES/iPS
(ReproCELL)

### <Conditions for Immersion in Preservation Solution and Thawing>

(5-i) Freezing Medium (trademark) for 20 seconds + thawing with medium at 37°C
(5-ii) Freezing Medium (trademark) for 1 minute + thawing with medium at 37°C
(5-iii) Freezing Medium (trademark) for 1 minute + thawing with 1 M Suc solution
(5-iv) StemCell Keep (trademark) for 20 seconds + thawing with medium at 37°C
(5-v) StemCell Keep (trademark) for 1 minute + thawing with medium at 37°C
(5-vi) StemCell Keep (trademark) for 1 minute + thawing with 1 M Suc solution
(5-vii) Control (no freezing)

RPE cells that had been cultured in a maintenance medium were trypsinized and recovered, and the cells were put in a centrifuge tube at a concentration of 1.0×10⁶ cells/tube, followed by suspension in a preservation solution. After immersion in liquid nitrogen, the cells were thawed in a warm bath at 37°C, followed by trypan blue staining to calculate the viable cell count.

As a result, in both cryopreservation solutions, the shorter the immersion time was, the higher the viable cell rate was. When the cryopreservation solutions were compared, StemCell Keep (trademark) showed a higher viable cell rate, and maintained about 90% of the control. The viable cell rate was higher when thawing was performed in a 1 M Suc solution rather than a medium.

### Example 6

Toxicity to RPE cells constituting a sheet could be suppressed by adopting a stepwise treatment as the pretreatment step and selecting a rapid cryopreservation solution as the preservation solution used in the step of immersing in a preservation solution, but occurrence of damage to the sheet structure was a problem. Specifically, when an RPE cell sheet was frozen by vitrification and thawed and then SYTOX (trademark) Green staining was performed, a narrow linear or concentric distribution of dead cells was observed. The distribution of the dead cells is clearly different from the distribution of SYTOX (trademark)-positive cells as observed in the toxicity of a cryopreservation solution, and is considered as a shape due to various causes. When parts of the SYTOX (trademark)-positive cells were observed, they were in a state in which the sheet was shriveled, and an appearance in which the dead cells were compressed and crushed (aggregate of linear dead cells: fold) was observed. It was guessed that this fold is structural damage due to a volume change in which the sheet contracts during vitrification freezing, and the freezing step was improved.

As the freezing methods, a metal contact method in which the freezing speed is excellent and vapor phase freezing for the purpose of reducing the speed during vitrification freezing were investigated. During vapor phase freezing, the height from the liquid level of liquid nitrogen in a vapor phase was changed, and the speed of vapor phase freezing was set at two grades. As a test specimen, in terms of confirming the freezing and thawing resistance of the RPE cell sheet itself, a single RPE cell sheet having no collagen gel that is usually used as a scaffold for culture of an RPE cell sheet was used.

### <Test Conditions>

Specimen: culture substrate-coated RPE cell sheet (CELLStart (trademark), Life Technologies, A10142-01)
Pretreatment solution: 5-serially diluted solutions (10% diluted solution, 20% diluted solution, 30% diluted solution, 40% diluted solution and 50% diluted solution) of 50%(v/v) StemCell Keep (trademark) (Bioverde) (theoretical composition: EG20%, Suc 0.375 M and COOH-PLL 5%)
Cryopreservation solution: StemCell Keep (trademark) (Bioverde) Step flow: pretreatment step -> step of immersing in preservation solution -> freezing step -> -130°C for 10 minutes -> thawing step

### (Fabrication of RPE Cell Sheet)

The inside of a Transwell (trademark) (Corning, #3460) insert was coated with an aqueous protein substrate solution CELLStart (trademark). As the aqueous protein substrate solution, a solution obtained by diluting 20 µL of CELLStart (trademark) with 980 µL of PBS(+) (SIGMA, D8662) was used, and 300 µL of this aqueous protein substrate solution was added to the inside of the insert. After allowing to stand in an incubator at 37°C for 30 minutes, those obtained by removing the supernatant of the aqueous protein substrate solution were used as a culture substrate-coated insert. Into the inside of the insert coated with the aqueous protein substrate solution, RPE cells (manufactured by Lonza K.K., 00194987) were seeded so that the number of cells were 5.0 × 10⁵ (F-10 medium supplemented with 10(v/v)% FBS, 500 µL), and 1,500 µL of the F-10 medium supplemented with 10(v/v)% FBS was added to the outside of the insert. Until the cells reached confluent, culture was performed in the F-10 medium supplemented with 10(v/v)% FBS, and when the cells reached confluent, the medium was replaced with the above-mentioned maintenance medium, and culture was continued. Medium replacement was performed twice to three times/week.

### Pretreatment Step

Of the StemCell Keep (trademark) (Bioverde) serially diluted solutions, 500 µL of the 10% diluted solution was added to the inside of the Transwell (trademark) insert and 1,000 µL of the same was added to the outside of the insert, followed by allowing to stand at room temperature for 2 minutes. Then, the StemCell Keep (trademark) (Bioverde) serially diluted solutions were replaced in order, and the concentration was increased to 50%, followed by a stepwise dehydration treatment for 2 minutes × 5 times.

### Step of Immersing in Preservation Solution

The diluted solution was replaced with StemCell Keep (trademark) (Bioverde) (0°C) of the same volume, followed by allowing to stand for 30 seconds to 5 minutes while cooling in ice.

### Freezing Step and Thawing Step

Then, the StemCell Keep (trademark) (Bioverde) was removed, and the RPE cell sheet was frozen by liquid nitrogen vapor phase freezing or the metal contact method (copper plate).

In the vapor phase freezing, the distance from the liquid level of liquid nitrogen was set at about 5 mm above, and a freezing treatment was performed.

Change in the distance from the liquid level of liquid nitrogen was made by adjusting the height of a PTFE membrane filter (POREFLON WP-500-100, manufactured by Sumitomo Electric Industries, Ltd.) mounted on a float for freezing. Here, the float for freezing refers to that in which styrofoam is formed into a doughnut form and the cavity in the central part is covered with the PTFE membrane filter, as shown in FIG. 4. Liquid nitrogen in a vapor phase passes through the cavity of the central part, and is brought into contact with an RPE cell sheet arranged together with a Transwell (trademark) insert on the membrane filter, thus enabling freezing. By adjusting the height at which this membrane filter is fixed, the height from the liquid level of liquid nitrogen in a vapor phase at which freezing is performed was set.

The RPE cell sheet was allowed to stand for 10 minutes, and subjected to a freezing treatment.

Then, using a temperature keeper, the RPE cell sheet was allowed to stand for 10 minutes at -130°C. This temperature keeper is an apparatus that sets such that the temperature of an aluminium plate on which an insert of Transwell (trademark) including the RPE cell sheet is arranged is maintained at -130°C by a temperature controller. The temperature set by the temperature controller was measured with the thermocouple mentioned below. Then, the Transwell (trademark) insert was quickly put in 100 mL of a 1 M Suc solution at 37°C, followed by immersion for 1 minute to perform thawing. Then, 500 uL of a 0.5 M Suc solution was put in the inside of the Transwell (trademark) insert and 1,000 uL of the same was put in the outside of the insert at room temperature to perform replacement, followed by allowing to stand for 3 minutes. Then, replacement with a maintenance medium of the same volume at room temperature was performed, and allowing to stand for 5 minutes was repeated twice. Then, the medium was replaced with a maintenance medium of the same volume, followed by incubation at 37°C. The frozen and thawed RPE cell sheet was further cultured for 2 weeks.

### Method for Measuring TER Value

The probes of Millicell ERS-2 (Millipore) were washed with 70(v/v)% ethanol, followed by a maintenance medium, to perform equilibration. Then, the shorter probe was vertically inserted into the inside of the insert of Transwell (trademark) of the RPE cell sheet to be measured and the longer probe was vertically inserted into the outside of the insert, and after the measurement value became lowest and stable, the measurement value was recorded. The resistance value of the insert of Transwell (trademark) of only collagen gel was measured in the same manner, and this value was used as a blank and subtracted from the measurement value of the RPE cell sheet to obtain the TER value of the specimen.

### Method for Measuring Temperature with Thermocouple

Using a metal E thermocouple (wire diameter of 0.32 mm: positive electrode (alloy mainly made of nickel and chromium): negative electrode (alloy mainly made of copper and nickel)), the measured temperature was monitored with a data logger (HIOKI, LR8431). The thermocouple was arranged in the part to be measured, and after the temperature became stable, the temperature was recorded in a stable state.

The results were as shown in FIG. 5A to FIG. 5C.

As shown in FIG. 5A, in both of metal contact and vapor phase freezing, complete vitrification was confirmed. Both cell sheets by metal contact and vapor phase freezing maintained the cell morphology and the sheet structure before thawing at both of immediately after thawing and during further culture (after 120 hours).

As shown in FIG. 5B, when dead cells of the RPE cell sheet after thawing were stained with SYTOX (trademark) Green, a narrow linear or concentric distribution of dead cells was observed in the metal contact method (region represented by a dotted line), but the sheet that was subjected to vapor phase freezing at 5 mm above the liquid level of liquid nitrogen showed no distribution of dead cells.

As shown in FIG. 5C, TER values were measured before and after thawing, the sheet having no fold showed rapid recovery of the TER value, and the TER value recovered to the TER value before freezing in 2 weeks of further culture. The TER value was at a level at which the sheet can be sufficiently used as a transplantation material.

Meanwhile, the sheet having a fold showed recovery of the TER value, but the recovery rate was lower than that of the sheet having no fold, and the TER value could not recover to the TER value before freezing by further culture for 2 weeks.

The above-mentioned results confirmed that the RPE cell sheet can be frozen and thawed, and can maintain the cell morphology and the sheet structure. It is considered that, by solving the fold, recovery of the sheet after thawing can be made more rapid.

### Example 7

### 7-1

In Example 6, it was confirmed that, even when an RPE cell sheet having no collagen gel that is usually used as a scaffold is thawed, the cell morphology and the sheet structure can be maintained. Meanwhile, in the case of an RPE cell sheet having no collagen gel as a scaffold, the RPE cell sheet cannot be recovered by collagenase, and there is no means of isolating the RPE cell sheet from the insert when used in transplantation treatment.

Thus, thawing of an RPE cell sheet having a collagen gel as a scaffold was investigated.

The collagen gel as a scaffold generally has a meniscus structure and in a state in which the central part is thin and the margin is thick, and in the insert for culture, the margin has a thickness of about 1 to 2 mm. The results of a preliminary test (as the freezing method, the metal contact method was adopted) performed according to the method of Example 6 revealed that, when a collagen gel having such thickness is completely vitrified, it requires 15 to 30 minutes as the immersion time in a preservation solution.

In terms of reducing the toxicity to RPE cells, it is preferable to avoid immersing in a preservation solution for a long period of time. Thus, compatibility of complete vitrification during freezing with recovery of a sheet by collagenase by thinning (making into a thin film) a collagen gel was subsequently investigated.

### 7-2

### Investigation of Method for Thinning Collagen Gel

As the causes of the impossibility of complete vitrification of a conventional collagen gel, (i) thickness of the margin of the gel due to the meniscus structure and (ii) thickness of the whole gel are considered. Thus, in order to solve these causes, a method for thinning a collagen gel was validated.

### Air Drying Method

A 0.21(w/v)% collagen gel solution was added to Transwell (trademark) (bottom membrane diameter of 12 mm, culture area of 1.12 cm², Corning, #3460, 12-well plate) (gel amount: 200 µL), followed by incubation at 37°C for 30 minutes to fabricate a collagen gel. Thereafter, the collagen gel thus fabricated was air dried overnight in a clean bench (UV off) with the lid of the plate being closed. After drying, the presence or absence of cracks on the surface of the gel was confirmed. Then, the collagen gel was swelled in a culture medium, followed by allowing to stand in an incubator at 37°C overnight. As a result, many grooves occurred on the gel surface during drying, and the meniscus structure of the swelled gel could not be solved.

### Centrifugation Method

A 0.21(w/v)% collagen gel solution was added to Transwell (trademark) (bottom membrane diameter of 12 mm, culture area of 1.12 cm², Corning, #3460, 12-well plate) (gel amount: 200 µL), followed by incubation at 37°C for 30 minutes. Then, the Transwell (trademark) was vertically put in a 50 mL tube, followed by a centrifugal treatment (1,500 × g, room temperature, 2 hours and 20 minutes). In the centrifugal treatment, the angle was changed every 20 minutes so that no imbalance of the gel occurred. Using the collagen gel layer thus obtained, a treatment was performed in accordance with the description in Example 6. As a result, in some cases, the thickness of the gel remained, and complete vitrification could not be performed. Many grooves had occurred on the gel surface after drying.

### Centrifugation Method + Air Drying Method

A 0.21(w/v)% collagen gel solution was added to Transwell (trademark) (bottom membrane diameter of 12 mm, culture area of 1.12 cm², Corning, #3460, 12-well plate) (gel amount: 200 µL), followed by incubation at 37°C for 30 minutes. Then, the Transwell (trademark) was vertically put in a 50 mL tube, followed by a centrifugal treatment (1,500 × g, room temperature, 2 hours and 20 minutes). In the centrifugal treatment, the angle was changed every 20 minutes so that no imbalance of the gel occurred. The collagen gel solution thus obtained after the centrifugal treatment was air dried overnight in a clean bench (UV off) with the lid of the plate being closed. After drying, the presence or absence of cracks on the surface of the gel was confirmed. Then, the collagen gel was swelled in a culture medium, followed by allowing to stand in an incubator at 37°C overnight. Then, the media in the inside and outside of the insert were replaced, and RPE cells were seeded on the collagen gel thus obtained, followed by culture until the color and the morphology of the RPE cells became appropriate.

As a result, a laminate of a collagen gel that was made into a thin film having a mean thickness of 20 to 50 µm and an RPE cell sheet was obtained. In the measurement of the thickness of the RPE cell sheet, an inverted microscope equipped with a motorized stage (manufactured by Olympus Corporation, IX83) was used. Specifically, first, the center on the cell surface of the RPE cell sheet and four points on the sheet margin (when an X-axis and a Y-axis that are orthogonal in the center are assumed, four points of intersection of the X-axis and Y-axis and the sheet margin) were used as measurement points. Then, at these measurement points, the Z coordinate on the membrane surface was subtracted from the Z coordinate on the cell surface of the RPE cell sheet, and the mean value of the numerical values thus obtained was regarded as the thickness.

### 7-3

### Investigation of Vitrification of Laminate of Collagen Gel and RPE Cell Sheet

In accordance with the method mentioned in Example 6, regarding the laminate of the thinned collagen gel and the RPE cell sheet obtained in 7-2, a pretreatment step, a step of immersing in a preservation solution, a freezing step, a step of allowing to stand at -130°C for 10 minutes and a thawing step were performed. As the freezing method, vapor phase freezing (liquid nitrogen (liquid level of LN₂ of 0.5 cm) was used, and the duration of the step of immersing in a preservation solution was 30 seconds.

The photograph after freezing was as shown in FIG. 6. It was confirmed that the laminate of the collagen gel and the RPE cell sheet is completely vitrified.

It was confirmed that, when the RPE cell sheet on the laminate of the collagen gel and the RPE cell sheet is recovered, the collagen gel is degraded by collagenase, and only the sheet can be recovered.

### Example 8

### 8-1: Investigation of Freezing Method in Laminate of Thinned

### Collagen Gel and RPE Cell Sheet

Regarding the laminate of the thinned collagen gel and the RPE cell sheet obtained in 7-2, in accordance with the method of Example 7, thawing tests were performed under various conditions, and the thawing conditions for complete vitrification when the thinned collagen gel is used were investigated.

As the freezing methods, 1) a metal contact method (copper plate) (-192°C), 2) vapor phase freezing at the liquid level of liquid nitrogen (LN₂), 3) vapor phase freezing at about 10 mm above the liquid level of LN₂, 4) vapor phase freezing at about 25 mm above the liquid level of LN₂, 5) vapor phase freezing at about 40 mm above the liquid level of LN₂, and 6) vapor phase freezing at about 80 mm above the liquid level of LN₂ were adopted.

When the temperature was measured using an E thermocouple (wire diameter of 0.32 mm: data logger LR8431 (HIOKI) was used, measurement interval of 100 ms), the relationship between the distance from the liquid level of liquid nitrogen and the temperature was as follows. The positive electrode of the E thermocouple was an alloy made of nickel and chromium, the negative electrode was an alloy made of copper and nickel, and the measurement range was -200 to +900°C.

Liquid level: -191°C

5 mm above the liquid level: -185°C
10 mm above the liquid level: -180°C
30 mm above the liquid level: -150°C
40 mm above the liquid level: -132°C
60 mm above the liquid level: -114°C
80 mm above the liquid level: -105°C

The results of observation of cell morphology by microscopy were as shown in Table 1. At 80 mm above LN₂ (freezing temperature of -105°C), the cell morphology was not maintained.

**[Table 1]**

| Freezing condition | Temperature (°C) | Cell morphology |
|---|---|---|
| Metal contact | -192 | Morphology was maintained |
| Liquid level of LN₂ | -191 | Morphology was maintained |
| 10 mm above LN₂ | -180 | Morphology was maintained |
| 25 mm above LN₂ | N.D.^{∗} | Morphology was maintained |
| 40 mm above LN₂ | -132 | Morphology was maintained |
| 80 mm above LN₂ | -105 | Morphology was not maintained |

| | | |
|---|---|---|
| N.D.) Not determined. | | |

The results of observation of the fold formation and the vitrification state after dead cells of the RPE cell sheet after thawing were stained with SYTOX (trademark) Green were as shown in Table 2.

**[Table 2]**

| Freezing condition | Temperature (°C) | Vitrification | Fold formation |
|---|---|---|---|
| Metal contact | -192 | Whole | Many |
| Liquid level of LN₂ | -191 | Whole | Only a few** |
| 10 mm above LN₂ | -180 | Whole | None |
| 25 mm above LN₂ | N.D.^{∗} | Whole | None |
| 40 mm above LN₂ | -132 | Whole | None |
| 80 mm above LN₂ | -105 | Partial | None |

| | | | |
|---|---|---|---|
| ^{∗} N.D.) Not determined. ^{∗∗} Only a few) Although short folds were confirmed, occurrence of folds were suppressed compared with the metal contact. | | | |

As shown in FIG. 7, in the metal contact method (copper plate), a narrow linear or concentric distribution of dead cells (fold) was confirmed. Meanwhile, in vapor phase freezing (freezing at 10 to 80 mm above the liquid level of liquid nitrogen), no fold was confirmed. Particularly, in vapor phase freezing (freezing at 10 to 40 mm above the liquid level of liquid nitrogen), complete vitrification was also confirmed over the whole sheet.

### 8-2: Confirmation of Temperature Fall Rate in Laminate of Thinned

### Collagen Gel and RPE Cell Sheet

The temperature change of a collagen gel (150 µL of thinned collagen gel) during vitrification freezing was measured with a thermocouple, and the temperature fall rate at 10 to -40°C was investigated.

When the temperature was measured using a K thermocouple (wire diameter of 0.05 mm: data logger LR8431 (HIOKI) was used, measurement interval of 100 ms), the relationship between the distance from the liquid level of liquid nitrogen and the temperature was as follows. The positive electrode of the K thermocouple was an alloy made of nickel and chromium, the negative electrode was an alloy made of nickel and aluminum, and the measurement range was -200 to +1,200°C.

Specifically, 1) the thermocouple was arranged such that the measurement points of the thermocouple were in contact with the surface of the collagen gel in the insert.
2) The fact that the temperature before freezing was room temperature was confirmed, and measurement of the temperature was started. While the thermocouple was in a state of being contact with the collagen gel, the collagen gel in the insert was subjected to vitrification freezing by each freezing method.
3) After freezing, the collagen gel was allowed to stand until the temperature fell and almost no temperature change was observed, and the temperature data were recorded.
4) At the time point of 3 minutes after no temperature change, the measurement was completed.
5) Data were recovered from the data logger, and the time required when the temperature fell from 10°C to -40°C was calculated, thus calculating the temperature fall rate.

As a result, the temperature fall rate was 1) 145°C/second in the metal contact method, 2) 45.7°C/second in vapor phase freezing at the liquid level of liquid nitrogen (LN₂), and 3) 22.7°C/second in vapor phase freezing at about 10 mm above the liquid level of LN₂.

### 8-3: Investigation of Immersion Time in Preservation Solution in

### Laminate of Thinned Collagen Gel and RPE Cell Sheet

Then, in order to minimize the cytotoxicity, the immersion time in a preservation solution was investigated in accordance with the method mentioned in Example 7. The immersion time in a preservation solution was set at 0.01 to 30 seconds. Here, immersion for "0.01 second" means removal immediately after addition of a preservation solution. As the freezing method, vapor phase freezing (freezing at 10 mm above the liquid level of liquid nitrogen) was used. The time to transition from the step of immersing in a preservation solution to the freezing step was about 20 seconds.

The results were as shown in FIG. 8.

Under both conditions of immersion in a preservation solution for 0.01 second and 30 seconds, complete vitrification of the laminate could be confirmed. When immersion in a preservation solution for 0.01 second was compared with that for 30 seconds, in immersion for 0.01 second, the number of SYTOX (trademark) Green-positive cells was smaller, and cell detachment on the sheet margin was less. Regarding the RPE cell sheet that was subjected to a thawing treatment under the condition of immersion in a preservation solution for 0.01 second, the sheet structure of the central part of the sheet was maintained, and even after further culture, maintenance of the cell morphology and the sheet structure could be confirmed.

From the above-mentioned results, it was confirmed that even under the condition of immersion in a preservation solution for about 0.01 second as the condition for freezing the laminate, at least cytotoxicity and cell detachment can be suppressed, and complete vitrification is possible.

### Example 9

### 9-1

The results of the preliminary experiment revelated that, in the case of applying the methods mentioned in Patent Document 1 and Non-Patent Document 1 to an RPE cell sheet, when the RPE cell sheet is thawed, cracks may occur in the RPE cell sheet. The cracks occur linearly or circularly, and they are structural damage in which cracks occur in the collagen gel as a scaffold together with the RPE cell sheet. Generally, when cracks occur, cells in the crack part (border region) of the sheet after thawing are dead, and spread of cell detachment from the periphery of the cracks during recovery culture is observed. If cracks occur, maintenance of the sheet structure after thawing becomes impossible, and therefore, in the thinned collagen gel and the RPE cell sheet obtained in Example 7, the conditions in which no cracks occur were investigated as mentioned below.

It can be confirmed that cracks generally occur immediately after thawing, and a breaking sound during the thawing step is heard, and thus it was predicted that cracks occur at this timing. It was hypothesized that a volume change, which is a cause of cracks, may occur when the glass transition temperature is passed through at the timing of devitrification during thawing. Thus, whether cracks occur when a frozen body of an RPE cell sheet is thawed by slowly passing through the glass transition temperature (near -130°C) of water was validated.

Specifically, in accordance with the method mentioned in Example 6, the laminate of the thinned collagen gel and the RPE cell sheet obtained in Example 7 was allowed to stand at -130°C for 10 to 30 minutes before immersing in a thawing solution.

The results were as shown in Table 3.

**[Table 3]**

| Freezing condition | Presence or absence of cracks after freezing | Cell death at the periphery of cracks |
|---|---|---|
| Not kept at -130°C | Present | Present |
| Kept at -130°C for 10 minutes | Absent | Absent |
| Kept at -130°C for 20 minutes | Absent | Absent |
| Kept at -130°C for 30 minutes | Absent | Absent |

In all of the samples kept at about -130°C for 10 to 30 minutes before immersing in a thawing solution, no cracks occurred (FIG. 9). It was inferred that occurrence of cracks during thawing of the RPE cell sheet is likely to be due to a volume change because of a too fast thawing speed when the glass transition temperature is passed through. It is considered that cracks can be solved and the sheet structure can be maintained by adopting, after freezing, a step of keeping at a low temperature, at which effects of a volume change are small, and then thawing.

### 9-2

The temperature range in which cracks that occur during thawing of the RPE cell sheet can be solved was investigated by the same method as in 10-2, except that the temperature at which keeping is performed for 10 to 30 minutes before immersing in a thawing solution during thawing was -170°C, -150°C, -140°C, -130°C, -120°C or -100°C. As a comparative example, thawing was performed without keeping at a constant temperature (direct thawing).

As a result, as shown in FIG. 10, cracks occurred in direct thawing and at -170°C and -100°C, while no cracks occurred at -150°C, -140°C, -130°C and -120°C.

## Claims

1. A method for producing a frozen body of a three-dimensional tissue aggregate of retinal pigment epithelial cells, the method comprising:
a step of preparing the three-dimensional tissue aggregate of the retinal pigment epithelial cells, and
a step of subjecting the three-dimensional tissue aggregate of the retinal pigment epithelial cells to a freezing treatment at a predetermined freezing temperature set in advance using, as an index, the occurrence rate of a linear aggregate of dead cells in the three-dimensional tissue aggregate of the retinal pigment epithelial cells after thawing, thus obtaining the frozen body.

2. The method according to claim 1, wherein the freezing temperature is -191°C to -105°C.

3. The method according to claim 1 or 2, wherein the freezing treatment is performed in a vapor phase.

4. The method according to claim 3, wherein the vapor phase comprises liquid nitrogen gas.

5. The method according to any one of claims 1 to 4, wherein the freezing treatment is performed by a vitrification freezing method.

6. The method according to any one of claims 1 to 5, the method comprising a step of adjusting a freezing temperature of the three-dimensional tissue aggregate of the retinal pigment epithelial cells using, as an index, the occurrence rate of a linear aggregate of dead cells in the three-dimensional tissue aggregate of the retinal pigment epithelial cells after thawing.

7. The method according to any one of claims 1 to 6, wherein the temperature fall rate in the freezing treatment is 1.5°C/second to 144°C/second.

8. The method according to any one of claims 1 to 7, wherein the step of preparing the three-dimensional tissue aggregate comprises a step of subjecting the retinal pigment epithelial cells to a dehydration treatment.

9. The method according to claim 8, wherein the dehydration treatment comprises a step of bringing the retinal pigment epithelial cells into contact with a dehydrating agent.

10. The method according to claim 8 or 9, wherein the dehydration treatment is performed a plurality of times.

11. The method according to claim 9 or 10, wherein the dehydrating agent comprises polyhydric alcohol.

12. The method according to any one of claims 9 to 11, which increases the concentration of the dehydrating agent in each dehydration treatment over time.

13. The method according to claim 11 or 12, wherein the polyhydric alcohol comprises ethylene glycol.

14. The method according to any one of claims 1 to 13, wherein the step of preparing the three-dimensional tissue aggregate comprises a step of immersing the retinal pigment epithelial cells in a preservation solution after the dehydration treatment.

15. The method according to claim 14, wherein the immersion time is 0.01 to 60 seconds.

16. The method according to claim 14 or 15, wherein the preservation solution comprises at least a cryoprotective agent.

17. The method according to any one of claims 1 to 16, wherein the three-dimensional tissue aggregate is arranged on a carrier layer.

18. The method according to any one of claims 1 to 17, wherein the step of preparing the three-dimensional tissue aggregate comprises a step of seeding the retinal pigment epithelial cells on the carrier layer to culture them.

19. The method according to claim 17 or 18, wherein the carrier layer comprises collagen as a principal component.

20. The method according to any one of claims 17 to 19, wherein the mean thickness of the carrier layer is 200 µm or less.

21. The method according to any one of claims 1 to 20, wherein the three-dimensional tissue aggregate of the retinal pigment epithelial cells is a sheet of the retinal pigment epithelial cells.

22. The method according to claim 21, wherein the sheet of the retinal pigment epithelial cells is a monolayer.

23. A frozen body of a three-dimensional tissue aggregate of retinal pigment epithelial cells, which is obtained by the method according to any one of claims 1 to 22.

24. A method for preserving a three-dimensional tissue aggregate of retinal pigment epithelial cells, the method comprising a step of preserving the frozen body according to claim 23 in a cryopreservation environment.

25. A method for transporting a three-dimensional tissue aggregate of retinal pigment epithelial cells, the method comprising a step of transporting the frozen body according to claim 23 in a cryopreservation environment.

26. A method for producing a three-dimensional tissue aggregate of retinal pigment epithelial cells, the method comprising a step of thawing the frozen body according to claim 23.

27. The method according to claim 26, wherein the step of thawing the frozen body comprises a step of keeping the frozen body at near the glass transition temperature of water.

28. The method according to claim 26 or 27, wherein the step of thawing the frozen body comprises a step of keeping the frozen body at -160°C to -110°C.

29. The method according to claim 27 or 28, wherein the time to keep the frozen body is 1 to 60 minutes.

30. The method according to any one of claims 26 to 29, wherein the step of thawing the frozen body comprises a step of keeping the frozen body in a thawing solution.

31. A method for transporting a three-dimensional tissue aggregate of retinal pigment epithelial cells, the method comprising a step of transporting the thawed product obtained in the step according to claim 26.
